# EUROPEAN PATENT SPECIFICATION

(11) **EP 2 401 621 B1**
(45) Date of publication and mention of the grant of the patent: **14.08.2013**
(21) Application number: 10705113.8
(22) Date of filing: 22.02.2010
(51) Int. Cl.: G01N 33/574

(54) **USE OF S-ERBB-3 AS A MARKER FOR CANCER**
VERWENDUNG VON S-ERBB-3 ALS KREBSMARKER
UTILISATION DE S-ERBB-3 COMME MARQUEUR DES CANCERS

(30) Priority: 24.02.2009 EP 09002586
(43) Date of publication of application: 04.01.2012
(73) Proprietor: Roche Diagnostics GmbH, 68305 Mannheim (DE); F. Hoffmann-La Roche AG, 4070 Basel (CH)
(72) Inventor: RUTZ, Sandra, 81377 München (DE); ECKERT, Bernhard, 82362 Weilheim (DE); HEISS, Peter, 83671 Benediktbeuern (DE)
(74) Representative: Burger, Alexander
(86) International application number: PCT/EP2010/001087
(87) International publication number: WO 2010/097186

(56) References cited:
- US-A1- 2008 274 115
- LIN SUE-HWA ET AL: "Soluble ErbB3 levels in bone marrow and plasma of men with prostate cancer." CLINICAL CANCER RESEARCH : AN OFFICIAL JOURNAL OF THE AMERICAN ASSOCIATION FOR CANCER RESEARCH 15 JUN 2008, vol. 14, no. 12, 15 June 2008 (2008-06-15) , pages 3729-3736, XP002522235 ISSN: 1078-0432
- SIEGEL P M ET AL: "Elevated expression of activated forms of Neu / ErbB -2 and ErbB -3 are involved in the induction of mammary tumors in transgenic mice: implications for human breast cancer" EMBO JOURNAL, OXFORD UNIVERSITY PRESS, SURREY, GB, vol. 18, no. 8, 15 April 1999 (1999-04-15) , pages 2149-2164, XP002131033 ISSN: 0261-4189
- LEE H ET AL: "ISOLATION AND CHARACTERIZATION OF FOUR ALTERNATE C-ERBB3 TRANSCRIPTS EXPRESSED IN OVARIAN CARCINOMA-DERIVED CELL LINES AND NORMAL HUMAN TISSUES" ONCOGENE, NATURE PUBLISHING GROUP, GB BASINGSTOKE, HANTS, vol. 16, no. 25, 25 June 1998 (1998-06-25) , pages 3243-3252, XP001087557 ISSN: 0950-9232
- DUFFY M J: "CLINICAL USES OF TUMOR MARKERS: A CRITICAL REVIEW" CRITICAL REVIEWS IN CLINICAL LABORATORY SCIENCES, CRC PRESS, BACA RATON, FL, US, vol. 38, no. 3, 1 June 2001 (2001-06-01), pages 225-262, XP009042383 ISSN: 1040-8363

### SEQUENCE LISTING

<110> Roche Diagnostics GmbH
   F. Hoffmann-La Roche AG
<120> Use of s-ErbB-3 as a marker for cancer
<130> 25942 WO
<150> EP09002586
   <151> 2009-02-24
<160> 11
<170> PatentIn version 3.3
<210> 1
   <211> 631
   <212> PRT
   <213> Homo sapiens
<400> 1
   Met Arg Ala Asn Asp Ala Leu Gln Val Leu Gly Leu Leu Phe Ser Leu
   1               5                   10                  15
   Ala Arg Gly Ser Glu Val Gly Asn Ser Gln Ala Val Cys Pro Gly Thr
               20                  25                  30
   Leu Asn Gly Leu Ser Val Thr Gly Asp Ala Glu Asn Gln Tyr Gln Thr
           35                  40                  45
   Leu Tyr Lys Leu Tyr Glu Arg Cys Glu Val Val Met Gly Asn Leu Glu
       50                  55                  60
   Ile Val Leu Thr Gly His Asn Ala Asp Leu Ser Phe Leu Gln Trp Ile
   65                  70                  75                  80
   Arg Glu Val Thr Gly Tyr Val Leu Val Ala Met Asn Glu Phe Ser Thr
                   85                  90                  95
   Leu Pro Leu Pro Asn Leu Arg Val Val Arg Gly Thr Gln Val Tyr Asp
               100                 105                 110
   Gly Lys Phe Ala Ile Phe Val Met Leu Asn Tyr Asn Thr Asn Ser Ser
           115                 120                 125
   His Ala Leu Arg Gln Leu Arg Leu Thr Gln Leu Thr Glu Ile Leu Ser
       130                 135                 140
   Gly Gly Val Tyr Ile Glu Lys Asn Asp Lys Leu Cys His Met Asp Thr
   145                 150                 155                 160
   Ile Asp Trp Arg Asp Ile Val Arg Asp Arg Asp Ala Glu Ile Val Val
                   165                 170                 175
   Lys Asp Asn Gly Arg Ser Cys Pro Pro Cys His Glu Val Cys Lys Gly
               180                 185                 190
   Arg Cys Trp Gly Pro Gly Ser Glu Asp Cys Gln Thr Leu Thr Lys Thr
           195                 200                 205
   Ile Cys Ala Pro Gln Cys Asn Gly His Cys Phe Gly Pro Asn Pro Asn
       210                 215                 220
   Gln Cys Cys His Asp Glu Cys Ala Gly Gly Cys Ser Gly Pro Gln Asp
   225                 230                 235                 240
   Thr Asp Cys Phe Ala Cys Arg His Phe Asn Asp Ser Gly Ala Cys Val
                   245                 250                 255
   Pro Arg Cys Pro Gln Pro Leu Val Tyr Asn Lys Leu Thr Phe Gln Leu
               260                 265                 270
   Glu Pro Asn Pro His Thr Lys Tyr Gln Tyr Gly Gly Val Cys Val Ala
           275                 280                 285
   Ser Cys Pro His Asn Phe Val Val Asp Gln Thr Ser Cys Val Arg Ala
       290                 295                 300
   Cys Pro Pro Asp Lys Met Glu Val Asp Lys Asn Gly Leu Lys Met Cys
   305                 310                 315                 320
   Glu Pro Cys Gly Gly Leu Cys Pro Lys Ala Cys Glu Gly Thr Gly Ser
                   325                 330                 335
   Gly Ser Arg Phe Gln Thr Val Asp Ser Ser Asn Ile Asp Gly Phe Val
               340                 345                 350
   Asn Cys Thr Lys Ile Leu Gly Asn Leu Asp Phe Leu Ile Thr Gly Leu
           355                 360                 365
   Asn Gly Asp Pro Trp His Lys Ile Pro Ala Leu Asp Pro Glu Lys Leu
       370                 375                 380
   Asn Val Phe Arg Thr Val Arg Glu Ile Thr Gly Tyr Leu Asn Ile Gln
   385                 390                 395                 400
   Ser Trp Pro Pro His Met His Asn Phe Ser Val Phe Ser Asn Leu Thr
                   405                 410                 415
   Thr Ile Gly Gly Arg Ser Leu Tyr Asn Arg Gly Phe Ser Leu Leu Ile
               420                 425                 430
   Met Lys Asn Leu Asn Val Thr Ser Leu Gly Phe Arg Ser Leu Lys Glu
           435                 440                 445
   Ile Ser Ala Gly Arg Ile Tyr Ile Ser Ala Asn Arg Gln Leu Cys Tyr
       450                 455                 460
   His His Ser Leu Asn Trp Thr Lys Val Leu Arg Gly Pro Thr Glu Glu
   465                 470                 475                 480
   Arg Leu Asp Ile Lys His Asn Arg Pro Arg Arg Asp Cys Val Ala Glu
                   485                 490                 495
   Gly Lys Val Cys Asp Pro Leu Cys Ser Ser Gly Gly Cys Trp Gly Pro
               500                 505                 510
   Gly Pro Gly Gln Cys Leu Ser Cys Arg Asn Tyr Ser Arg Gly Gly Val
           515                 520                 525
   Cys Val Thr His Cys Asn Phe Leu Asn Gly Glu Pro Arg Glu Phe Ala
       530                 535                 540
   His Glu Ala Glu Cys Phe Ser Cys His Pro Glu Cys Gln Pro Met Gly
   545                 550                 555                 560
   Gly Thr Ala Thr Cys Asn Gly Ser Gly Ser Asp Thr Cys Ala Gln Cys
                   565                 570                 575
   Ala His Phe Arg Asp Gly Pro His Cys Val Ser Ser Cys Pro His Gly
               580                 585                 590
   Val Leu Gly Ala Lys Gly Pro Ile Tyr Lys Tyr Pro Asp Val Gln Asn
           595                 600                 605
   Glu Cys Arg Pro Cys His Glu Asn Cys Thr Gln Gly Cys Lys Gly Pro
       610                 615                 620
   Glu Leu Gln Asp Cys Leu Gly
   625                 630
<210> 2
   <211> 331
   <212> PRT
   <213> Homo sapiens
<400> 2
   Met Arg Ala Asn Asp Ala Leu Gln Val Leu Gly Leu Leu Phe Ser Leu
   1               5                   10                  15
   Ala Arg Gly Ser Glu Val Gly Asn Ser Gln Ala Val Cys Pro Gly Thr
               20                  25                  30
   Leu Asn Gly Leu Ser Val Thr Gly Asp Ala Glu Asn Gln Tyr Gln Thr
           35                  40                  45
   Leu Tyr Lys Leu Tyr Glu Arg Cys Glu Val Val Met Gly Asn Leu Glu
       50                  55                  60
   Ile Val Leu Thr Gly His Asn Ala Asp Leu Ser Phe Leu Gln Trp Ile
   65                  70                  75                  80
   Arg Glu Val Thr Gly Tyr Val Leu Val Ala Met Asn Glu Phe Ser Thr
                   85                  90                  95
   Leu Pro Leu Pro Asn Leu Arg Val Val Arg Gly Thr Gln Val Tyr Asp
               100                 105                 110
   Gly Lys Phe Ala Ile Phe Val Met Leu Asn Tyr Asn Thr Asn Ser Ser
           115                 120                 125
   His Ala Leu Arg Gln Leu Arg Leu Thr Gln Leu Thr Glu Ile Leu Ser
       130                 135                 140
   Gly Gly Val Tyr Ile Glu Lys Asn Asp Lys Leu Cys His Met Asp Thr
   145                 150                 155                 160
   Ile Asp Trp Arg Asp Ile Val Arg Asp Arg Asp Ala Glu Ile Val Val
                   165                 170                 175
   Lys Asp Asn Gly Arg Ser Cys Pro Pro Cys His Glu Val Cys Lys Gly
               180                 185                 190
   Arg Cys Trp Gly Pro Gly Ser Glu Asp Cys Gln Thr Leu Thr Lys Thr
   195 200 205
   Ile Cys Ala Pro Gln Cys Asn Gly His Cys Phe Gly Pro Asn Pro Asn
   210 215 220
   Gln Cys Cys His Asp Glu Cys Ala Gly Gly Cys Ser Gly Pro Gln Asp
   225 230 235 240
   Thr Asp Cys Phe Ala Cys Arg His Phe Asn Asp Ser Gly Ala Cys Val
   245 250 255
   Pro Arg Cys Pro Gln Pro Leu Val Tyr Asn Lys Leu Thr Phe Gln Leu
   260 265 270
   Glu Pro Asn Pro His Thr Lys Tyr Gln Tyr Gly Gly Val Cys Val Ala
   275 280 285
   Ser Cys Pro His Asn Phe Val Val Asp Gln Thr Ser Cys Val Arg Ala
   290 295 300
   Cys Pro Pro Asp Lys Met Glu Val Asp Lys Asn Gly Leu Lys Met Cys
   305 310 315 320
   Glu Pro Cys Gly Gly Leu Cys Pro Lys Gly Gly
   325 330
<210> 3
   <211> 562
   <212> PRT
   <213> Homo sapiens
<400> 3
   Met Arg Ala Asn Asp Ala Leu Gln Val Leu Gly Leu Leu Phe Ser Leu
   1 5 10 15
   Ala Arg Gly Ser Glu Val Gly Asn Ser Gln Ala Val Cys Pro Gly Thr
   20 25 30
   Leu Asn Gly Leu Ser Val Thr Gly Asp Ala Glu Asn Gln Tyr Gln Thr
   35 40 45
   Leu Tyr Lys Leu Tyr Glu Arg Cys Glu Val Val Met Gly Asn Leu Glu
   50 55 60
   Ile Val Leu Thr Gly His Asn Ala Asp Leu Ser Phe Leu Gln Trp Ile
   65                  70                  75                  80
   Arg Glu Val Thr Gly Tyr Val Leu Val Ala Met Asn Glu Phe Ser Thr
                   85                  90                  95
   Leu Pro Leu Pro Asn Leu Arg Val Val Arg Gly Thr Gln Val Tyr Asp
               100                 105                 110
   Gly Lys Phe Ala Ile Phe Val Met Leu Asn Tyr Asn Thr Asn Ser Ser
           115                 120                 125
   His Ala Leu Arg Gln Leu Arg Leu Thr Gln Leu Thr Glu Ile Leu Ser
       130                 135                 140
   Gly Gly Val Tyr Ile Glu Lys Asn Asp Lys Leu Cys His Met Asp Thr
   145                 150                 155                 160
   Ile Asp Trp Arg Asp Ile Val Arg Asp Arg Asp Ala Glu Ile Val Val
                   165                 170                 175
   Lys Asp Asn Gly Arg Ser Cys Pro Pro Cys His Glu Val Cys Lys Gly
               180                 185                 190
   Arg Cys Trp Gly Pro Gly Ser Glu Asp Cys Gln Thr Leu Thr Lys Thr
           195                 200                 205
   Ile Cys Ala Pro Gln Cys Asn Gly His Cys Phe Gly Pro Asn Pro Asn
       210                 215                 220
   Gln Cys Cys His Asp Glu Cys Ala Gly Gly Cys Ser Gly Pro Gln Asp
   225                 230                 235                 240
   Thr Asp Cys Phe Ala Cys Arg His Phe Asn Asp Ser Gly Ala Cys Val
                   245                 250                 255
   Pro Arg Cys Pro Gln Pro Leu Val Tyr Asn Lys Leu Thr Phe Gln Leu
               260                 265                 270
   Glu Pro Asn Pro His Thr Lys Tyr Gln Tyr Gly Gly Val Cys Val Ala
           275                 280                 285
   Ser Cys Pro His Asn Phe Val Val Asp Gln Thr Ser Cys Val Arg Ala
   290 295 300
   Cys Pro Pro Asp Lys Met Glu Val Asp Lys Asn Gly Leu Lys Met Cys
   305 310 315 320
   Glu Pro Cys Gly Gly Leu Cys Pro Lys Ala Cys Glu Gly Thr Gly Ser
   325 330 335
   Gly Ser Arg Phe Gln Thr Val Asp Ser Ser Asn Ile Asp Gly Phe Val
   340 345 350
   Asn Cys Thr Lys Ile Leu Gly Asn Leu Asp Phe Leu Ile Thr Gly Leu
   355 360 365
   Asn Gly Asp Pro Trp His Lys Ile Pro Ala Leu Asp Pro Glu Lys Leu
   370 375 380
   Asn Val Phe Arg Thr Val Arg Glu Ile Thr Gly Tyr Leu Asn Ile Gln
   385 390 395 400
   Ser Trp Pro Pro His Met His Asn Phe Ser Val Phe Ser Asn Leu Thr
   405 410 415
   Thr Ile Gly Gly Arg Ser Leu Tyr Asn Arg Gly Phe Ser Leu Leu Ile
   420 425 430
   Met Lys Asn Leu Asn Val Thr Ser Leu Gly Phe Arg Ser Leu Lys Glu
   435 440 445
   Ile Ser Ala Gly Arg Ile Tyr Ile Ser Ala Asn Arg Gln Leu Cys Tyr
   450 455 460
   His His Ser Leu Asn Trp Thr Lys Val Leu Arg Gly Pro Thr Glu Glu
   465 470 475 480
   Arg Leu Asp Ile Lys His Asn Arg Pro Arg Arg Asp Cys Val Ala Glu
   485 490 495
   Gly Lys Val Cys Asp Pro Leu Cys Ser Ser Gly Gly Cys Trp Gly Pro
   500 505 510
   Gly Pro Gly Gln Cys Leu Ser Cys Arg Asn Tyr Ser Arg Gly Gly Val
   515 520 525
   Cys Val Thr His Cys Asn Phe Leu Asn Gly Tyr Ser Lys Gly Ser Gln
       530                 535                 540
   Ser Arg Met Gly Gly Gly Gly Ala Leu Gln Trp Asn Cys Ser Gly Gly
   545                 550                 555                 560
   Ile Gln
<210> 4
   <211> 534
   <212> PRT
   <213> Homo sapiens
<400> 4
   Met Arg Ala Asn Asp Ala Leu Gln Val Leu Gly Leu Leu Phe Ser Leu
   1               5                   10                  15
   Ala Arg Gly Ser Glu Val Gly Asn Ser Gln Ala Val Cys Pro Gly Thr
               20                  25                  30
   Leu Asn Gly Leu Ser Val Thr Gly Asp Ala Glu Asn Gln Tyr Gln Thr
           35                  40                  45
   Leu Tyr Lys Leu Tyr Glu Arg Cys Glu Val Val Met Gly Asn Leu Glu
       50                  55                  60
   Ile Val Leu Thr Gly His Asn Ala Asp Leu Ser Phe Leu Gln Trp Ile
   65                  70                  75                  80
   Arg Glu Val Thr Gly Tyr Val Leu Val Ala Met Asn Glu Phe Ser Thr
                   85                  90                  95
   Leu Pro Leu Pro Asn Leu Arg Val Val Arg Gly Thr Gln Val Tyr Asp
               100                 105                 110
   Gly Lys Phe Ala Ile Phe Val Met Leu Asn Tyr Asn Thr Asn Ser Ser
           115                 120                 125
   His Ala Leu Arg Gln Leu Arg Leu Thr Gln Leu Thr Glu Ile Leu Ser
       130                 135                 140
   Gly Gly Val Tyr Ile Glu Lys Asn Asp Lys Leu Cys His Met Asp Thr
   145                 150                 155                 160
   Ile Asp Trp Arg Asp Ile Val Arg Asp Arg Asp Ala Glu Ile Val Val
   165 170 175
   Lys Asp Asn Gly Arg Ser Cys Pro Pro Cys His Glu Val Cys Lys Gly
   180 185 190
   Arg Cys Trp Gly Pro Gly Ser Glu Asp Cys Gln Thr Leu Thr Lys Thr
   195 200 205
   Ile Cys Ala Pro Gln Cys Asn Gly His Cys Phe Gly Pro Asn Pro Asn
   210 215 220
   Gln Cys Cys His Asp Glu Cys Ala Gly Gly Cys Ser Gly Pro Gln Asp
   225 230 235 240
   Thr Asp Cys Phe Ala Cys Arg His Phe Asn Asp Ser Gly Ala Cys Val
   245 250 255
   Pro Arg Cys Pro Gln Pro Leu Val Tyr Asn Lys Leu Thr Phe Gln Leu
   260 265 270
   Glu Pro Asn Pro His Thr Lys Tyr Gln Tyr Gly Gly Val Cys Val Ala
   275 280 285
   Ser Cys Pro His Asn Phe Val Val Asp Gln Thr Ser Cys Val Arg Ala
   290 295 300
   Cys Pro Pro Asp Lys Met Glu Val Asp Lys Asn Gly Leu Lys Met Cys
   305 310 315 320
   Glu Pro Cys Gly Gly Leu Cys Pro Lys Ala Cys Glu Gly Thr Gly Ser
   325 330 335
   Gly Ser Arg Phe Gln Thr Val Asp Ser Ser Asn Ile Asp Gly Phe Val
   340 345 350
   Asn Cys Thr Lys Ile Leu Gly Asn Leu Asp Phe Leu Ile Thr Gly Leu
   355 360 365
   Asn Gly Asp Pro Trp His Lys Ile Pro Ala Leu Asp Pro Glu Lys Leu
   370 375 380
   Asn Val Phe Arg Thr Val Arg Glu Ile Thr Gly Tyr Leu Asn Ile Gln
   385 390 395 400
   Ser Trp Pro Pro His Met His Asn Phe Ser Val Phe Ser Asn Leu Thr
                   405                 410                 415
   Thr Ile Gly Gly Arg Ser Leu Tyr Asn Arg Gly Phe Ser Leu Leu Ile
               420                 425                 430
   Met Lys Asn Leu Asn Val Thr Ser Leu Gly Phe Arg Ser Leu Lys Glu
           435                 440                 445
   Ile Ser Ala Gly Arg Ile Tyr Ile Ser Ala Asn Arg Gln Leu Cys Tyr
       450                 455                 460
   His His Ser Leu Asn Trp Thr Lys Val Leu Arg Gly Pro Thr Glu Glu
   465                 470                 475                 480
   Arg Leu Asp Ile Lys His Asn Arg Pro Arg Arg Asp Cys Gly Glu Gly
                   485                 490                 495
   Lys Gly Leu Leu Gly Gly Glu Asn Arg Glu Ser Gly Arg Arg Gly Leu
               500                 505                 510
   Lys Gly Leu Phe Cys Pro Arg Arg Gly Ser Arg Val Glu Gly Trp Asn
           515                 520                 525
   Gln Gly Glu Gly Gly Cys
       530
<210> 5
   <211> 183
   <212> PRT
   <213> Homo sapiens
<400> 5
   Met Arg Ala Asn Asp Ala Leu Gln Val Leu Gly Leu Leu Phe Ser Leu
   1               5                   10                  15
   Ala Arg Gly Ser Glu Val Gly Asn Ser Gln Ala Val Cys Pro Gly Thr
               20                  25                  30
   Leu Asn Gly Leu Ser Val Thr Gly Asp Ala Glu Asn Gln Tyr Gln Thr
           35                  40                  45
   Leu Tyr Lys Leu Tyr Glu Arg Cys Glu Val Val Met Gly Asn Leu Glu
       50                  55                  60
   Ile Val Leu Thr Gly His Asn Ala Asp Leu Ser Phe Leu Gln Trp Ile
   65 70 75 80
   Arg Glu Val Thr Gly Tyr Val Leu Val Ala Met Asn Glu Phe Ser Thr
   85 90 95
   Leu Pro Leu Pro Asn Leu Arg Val Val Arg Gly Thr Gln Val Tyr Asp
   100 105 110
   Gly Lys Phe Ala Ile Phe Val Met Leu Asn Tyr Asn Thr Asn Ser Ser
   115 120 125
   His Ala Leu Arg Gln Leu Arg Leu Thr Gln Leu Thr Gly Gln Phe Pro
   130 135 140
   Met Val Pro Ser Gly Leu Thr Pro Gln Pro Ala Gln Asp Trp Tyr Leu
   145 150 155 160
   Leu Asp Asp Asp Pro Arg Leu Leu Thr Leu Ser Ala Ser Ser Lys Val
   165 170 175
   Pro Val Thr Leu Ala Ala Val
   180
<210> 6
   <211> 40
   <212> PRT
   <213> Homo sapiens
<400> 6
   Tyr Lys Tyr Pro Asp Val Gln Asn Glu Cys Arg Pro Cys His Glu Asn
   1 5 10 15
   Cys Thr Gln Gly Cys Lys Gly Pro Glu Leu Gln Asp Cys Leu Gly Gln
   20 25 30
   Thr Leu Val Leu Ile Gly Lys Thr
   35 40
<210> 7
   <211> 30
   <212> PRT
   <213> Homo sapiens
<400> 7
   Cys Gln Pro Cys Pro Ile Asn Cys Thr His Ser Cys Val Asp Leu Asp
   1 5 10 15
   Asp Lys Gly Cys Pro Ala Glu Gln Arg Ala Ser Pro Leu Thr
   20 25 30
<210> 8
   <211> 36
   <212> DNA
   <213> Artificial
<220>
   <223> Primer
<400> 8
   gcaagctagc caucatgagg gcgaacgacg ctctgc 36
<210> 9
   <211> 43
   <212> DNA
   <213> Artificial
<220>
   <223> Primer
<400> 9
   gcaagcggcc gccccacctt tgggacatag tcccccacaa ggc 43
<210> 10
   <211> 42
   <212> DNA
   <213> Artificial
<220>
   <223> Primer
<400> 10
   gcaagcggcc gcttgtatgc cacctgaaca gttccattgc ag 42
<210> 11
   <211> 42
   <212> DNA
   <213> Artificial
<220>
   <223> Primer
<400> 11
   gcaagcggcc gcacaccccc ttcctccttg gttccatccc tc 42

## Description

The present invention relates to a method aiding in the assessment of cancer. It discloses the use of s-ErbB-3 as a universal marker of different cancer types. Measurement of s-ErbB-3 can, e.g., be used in the early detection or diagnosis of cancer or in the surveillance of patients who undergo surgery.

Cancer remains a major public health challenge despite progress in detection and therapy. Cancer cells are characterized by the production of cancer-associated marker proteins. Cancer-associated proteins are found both in the tissues and in the bodily fluids of an individual who carries cancer cells. Their levels usually are low at the early stages of the carcinogenic progress and increase during the disease's progression and only in rare cases proteins are observed showing a decreased level in the course of disease progression. The sensitive detection of these proteins is an advantageous and promising approach for the diagnosis of cancer, in particular in an early stage diagnosis of cancer. The most prevalent cancer types are breast cancer (BC), lung cancer (LC) and colorectal cancer (CRC).

The most important therapeutic approaches for solid tumors are:
a) surgical resection of the tumor,
b) chemotherapy,
c) radiation therapy,
d) treatment with biologicals, like anti-tumor antibodies or anti-angiogenic antibodies and
e) a combination of the above methods.

Surgical resection of the tumors is widely accepted as a first line treatment for early stage solid tumors. Most cancers, however, are detected only when they become symptomatic, i.e. when patients already are in a rather late stage of disease progression.

The staging of cancer is the classification of the disease in terms of extent, progression, and severity. It groups cancer patients so that generalizations can be made about prognosis and the choice of therapy.

The different stages of BC or CRC used to be classified according to Dukes' stages A to D. Today, the TNM system is the most widely used classification of the anatomical extent of cancer. It represents an internationally accepted, uniform staging system. There are three basic variables: T (the extent of the primary tumor), N (the status of regional lymph nodes) and M (the presence or absence of distant metastases). The TNM criteria are published by the UICC (International Union Against Cancer), Sobin, L.H., Wittekind, Ch. (eds): TNM Classification of Malignant Tumours, sixth edition, 2002). Once the TNM status is determined the patients are grouped into disease stages that are denoted by Roman numerals ranging form I to IV with IV being the most advanced disease stage. TNM staging and UICC disease stages correspond to each other as shown in the following Table taken from Sobin L.H. and Wittekind (eds.) supra.

### Interrelation of TNM staging and UICC disease stages

| **UICC disease stage** | **T staging** | **N staging** | **M staging** |
|---|---|---|---|
| Stage 0 | Tᵢₛ | N0 | M0 |
| Stage I | T1, T2 | N0 | M0 |
| Stage IIA | T3 | N0 | M0 |
| Stage IIB | T4 | N0 | M0 |
| Stage IIIA | T1, T2 | N1 | M0 |
| Stage IIIB | T3, T4 | N1 | M0 |
| Stage IIIC | Any T | N2 | M0 |
| Stage IV | Any T | Any N | M1 |

What is especially important is, that early diagnosis of cancer, e.g. of BC or CRC translates to a much better prognosis. In CRC malignant tumors of the colorectum arise from benign tumors, i.e. from adenoma. Therefore, best prognosis have those patients diagnosed at the adenoma stage. Patients diagnosed as early as in stage Tᵢₛ, N0, M0 or T1-3; N0; M0, if treated properly have a more than 90% chance of survival 5 years after diagnosis as compared to a 5-years survival rate of only 10% for patients diagnosed when distant metastases are already present.

Current detection methods including imaging methods, such as X-ray or nuclear resonance imaging in theory might at least partially be appropriate for use as a general screening tool. However, they are very costly and not affordable to health care systems for a general and broad use in mass screenings of large numbers of subjects, particularly for subjects without any tumor symptoms.

Thus, it is an object of the present invention to provide a simple and cost-efficient procedure of tumor assessments, e.g. to identify individuals suspect of having cancer. For this purpose, a general tumor marker which is detectable in body fluids, e.g. blood or serum or plasma or a panel of such markers, would be desirable.

A number of serum tumor markers are already in clinical use. For example the soluble 30 kDa fragment of cytoceratin 19 (CYFRA 21-1), carcinoembryogenic antigen (CEA), neuron-specific enolase (NSE), and squamous cell carcinoma antigen (SCC) are the most prominent LC markers. However, none of them meets the criteria for sensitivity and specificity required for a screening tool (Thomas, L., Labor und Diagnose, TH Books Verlagsgesellschaft, Frankfurt/Main, Germany (2000)).

In order to be of clinical utility, a new diagnostic marker as a single marker should be comparable to other markers known in the art, or better. Or, a new marker should lead to a progress in diagnostic sensitivity and/or specificity either if used alone or in combination with one or more other markers, respectively. The diagnostic sensitivity and/or specificity of a test is best assessed by its receiver-operating characteristics, which will be described in detail below.

Whole blood, serum or plasma are the most widely used sources of sample in clinical routine. The identification of an early tumor marker that would aid in the reliable cancer detection or provide early prognostic information could lead to a method that would greatly aid in the diagnosis and in the management of this disease. Therefore, an urgent clinical need exists to improve the in vitro assessment of cancer and in particular of BC. It is especially important to improve the early diagnosis of cancer, e.g. BC, since for patients diagnosed early on chances of survival are much higher as compared to those diagnosed at a progressed stage of disease.

With regard to BC as a public health problem, it is essential that more effective screening and preventative measures for BC will be developed.

The earliest detection procedures available at present for breast cancer involve using clinical breast examination and mammography. However, significant tumor size must typically exist before a tumor is palpable or can be detected by a mammogram. The density of the breast tissue and the age are important predictors of the accuracy of screening mammography. The sensitivity ranges from 63% in women with extremely dense breasts to 87% in women with almost entirely fatty breasts. The sensitivity increases with age from 69% in women of about 40 years of age to 83% in women 80 years and older (Carney, P.A. et al., Ann. Intern. Med. 138 (2003) 168-175). Only 20 - 25% of mammographically detected abnormalities that are biopsied prove to be malignant. The visualization of precancerous and cancerous lesions represents the best approach to early detection, but mammography is an expensive test that requires great care and expertise both to perform and in the interpretation of results (WHO, Screening for Breast Cancer, May 10, 2002; Esserman, L. et al., J. Natl. Cancer Inst. 94 (2002) 369-375).

In the recent years a tremendous amount of so-called breast specific or even so-called BC specific genes has been reported. The vast majority of the corresponding research papers or patent applications are based on data obtained by analysis of RNA expression patterns in cancer tissue versus a different tissue or an adjacent normal tissue, respectively. Such approaches may be summarized as differential mRNA display techniques.

As an example for data available from mRNA-display techniques, WO 00/60076 shall be mentioned and discussed. This application describes and claims more than two hundred isolated polynucleotides and the corresponding polypeptides as such, as well as their use in the detection of BC. However, it is general knowledge that differences on the level of mRNA are not mirrored by the level of the corresponding proteins. A protein encoded by a rare mRNA may be found in very high amounts and a protein encoded by an abundant mRNA may nonetheless be hard to detect and find at all (Chen, G. et al., Molecular and Cellular Proteomics 1 (2002) 304-313). This lack of correlation between mRNA-level and protein level is due to reasons like mRNA stability, efficiency of translation, stability of the protein, etc.

There also are recent approaches investigating the differences in protein patterns between different tissues or between healthy and diseased tissue in order to identify candidate marker molecules which might be used in the diagnosis of BC. Wulfkuhle, J.D. et al., Cancer Research 62 (2002) 6740-6749 have identified fifty-seven proteins which were differentially expressed between BC tissue and adjacent normal tissue. No data from liquid samples obtained from an individual are reported.

WO 02/23200 reports about twelve breast cancer-associated spots as found by surface-enhanced laser desorption and ionization (SELDI). These spots are seen more frequently in sera obtained from patients with BC as compared to sera obtained from healthy controls. However, the identity of the molecule(s) comprised in such spot, e.g. their sequence, is not known.

Nipple aspirate fluid (NAF) has been used for many years as a potential non-invasive method to identify breast cancer-specific markers. Kuerer et al. compared bilateral matched pair nipple aspirate fluids from women with unilateral invasive breast carcinoma by 2D gel electrophoresis (Kuerer, H.M. et al., Cancer 95 (2002) 2276-2282). 30 to 202 different protein spots were detected in the NAF of breasts suffering from breast carcinoma and not in the matched NAF of the healthy breasts. These spots were detected by a gel image analysis. But the identity of the protein spots is not known.

Despite the large and ever growing list of candidate protein markers in the field of BC, to date clinical/diagnostic utility of these molecules is not known. In order to be of clinical utility a new diagnostic marker as a single marker should be at least as good as the best single marker known in the art. Or, a new marker should lead to a progress in diagnostic sensitivity and/or specificity either if used alone or in combination with one or more other markers, respectively. The diagnostic sensitivity and/or specificity of a test is best assessed by its receiver-operating characteristics, which will be described in detail below.

At present, only diagnostic blood tests based on the detection of cancer antigen 15-3 (CA 15-3), a tumor-associated mucin, and carcinoembryonic antigen (CEA), a tumor associated glycoprotein, are available to assist diagnosis in the field of BC. CA 15-3 is usually increased in patients with advanced breast cancer. CA 15-3 levels are rarely elevated in women with early stage breast cancer (Duffy, M.J., Crit. Rev. Clin. Lab. Sci. 38 (2001) 225-262). Cancers of the ovary, lung and prostate may also raise CA 15-3 levels. Elevated levels of CA 15-3 may be associated with non-cancerous conditions, such as benign breast or ovary disease, endometriosis, pelvic inflammatory disease, and hepatitis. Pregnancy and lactation can also cause CA 15-3 levels to raise (National Cancer Institute, Cancer Facts, Fact Sheet 5.18 (1998) 1-5). The primary use of CEA is in monitoring colorectal cancer, especially when the disease has metastasized. However, a variety of cancers can produce elevated levels of CEA, including breast cancer.

Due to the lack of organ and tumor specificity, neither measurement of CA 15-3 nor measurement of CEA are recommended for screening of BC. These tumor markers are helpful diagnostic tools in follow-up care of BC patients (Untch, M. et al., J. Lab. Med. 25 (2001) 343-352).

CYFRA 21-1 is currently regarded to be the best of the presently known tumor markers for lung cancer. Even though not organ-specific it is predominantly found in lung tissue. Sensitivity of CYFRA 21-1 for lung cancer is described to be between 46-61% at a specificity of 95% towards other benign lung diseases. Increased serum levels of CYFRA 21-1 are also associated with pronounced benign liver diseases, renal insufficiency and invasive bladder cancer. CYFRA 21-1 testing is recommended for postoperative therapy surveillance.

CEA belongs to the group of carcinofetal antigens, usually produced during embryogenesis. CEA is not organ-specific and predominantly used for monitoring of colorectal cancer. Besides malignancies, also several benign diseases such as cirrhosis, bronchitis, pancreatitis and autoimmune diseases are associated with increased CEA serum levels. At 95% specificity towards benign lung diseases its sensitivity for lung cancer is reported to be 29-44%. The primary use of CEA is in monitoring colorectal cancer, especially when the disease has metastasized. However, a variety of cancers can produce elevated levels of CEA, including breast cancer. A preferred use of CEA is therapy surveillance of lung cancer.

CA 15-3 (cancer antigen 15-3), a tumor-associated mucin, is available to assist diagnosis in the field of BC. CA 15-3 is usually increased in patients with advanced breast cancer. CA 15-3 levels are rarely elevated in women with early stage breast cancer (Duffy, M.J., Crit. Rev. Clin. Lab. Sci. 38 (2001) 225-262). Cancers of the ovary, lung and prostate may also raise CA 15-3 levels. Elevated levels of CA 15-3 may be associated with non-cancerous conditions, such as benign breast or ovary disease, endometriosis, pelvic inflammatory disease, and hepatitis. Pregnancy and lactation can also cause CA 15-3 levels to raise (National Cancer Institute, Cancer Facts, Fact Sheet 5.18 (1998) 1-5).

CA 19-9 (carbohydrate antigen 19-9), a sialylated Lewis (a) antigen) on a glycolipid is a tumor marker for gastrointestinal cancers. It occurs in fetal gastric, intestinal and pancreatic epithelia. Low concentrations can also be found in adult tissue in the liver, lungs, and pancreas. There is no correlation between tumor mass and the CA 19-9 assay values Therefore the determination of CA 19-9 cannot be used for the early detection of pancreatic carcinoma. As the mucin is excreted exclusively via the liver, even slight cholestasis can lead to clearly elevated CA 19-9 serum levels in some cases. The marker is mainly used as an aid in the monitoring of disease status in those patients having confirmed pancreatic cancer (sensitivity 70-87%). 3-7% of the population have the Lewis a-negative/b-negative blood group configuration and are unable to express the mucin with the reactive determinant CA 19-9. This must be taken into account when interpreting the findings.

ErbB-2 (HER-2) stands for "Human Epidermal growth factor Receptor 2" and an overexpression of the human c-erbB-2 gene functionally relates to higher aggressiveness in breast cancers. It is a member of the ErbB protein family, more commonly known as the epidermal growth factor receptor family (Peles, E. et al., Cell 69 (1992) 205-216). ErbB-2 (HER-2) has also been designated as CD340 (cluster of differentiation 340) or p185. ErbB-2 (HER-2) is notable for its role in the pathogenesis of breast cancer and as a target of treatment. It is a cell membrane surface-bound receptor tyrosine kinase and is normally involved in the signal transduction pathways leading to cell growth and differentiation. ErbB-2 is thought to be an orphan receptor, with none of the EGF family of ligands able to activate it. However, ErbB receptors dimerise on ligand binding, and ErbB-2 is the preferential dimerisation partner of other members of the ErbB family. The human c-erbB-2 gene is a proto-oncogene located at the long arm of human chromosome 17(17q11.2-q12).

In the sense of the present invention early diagnosis of BC refers to a diagnosis at a pre-cancerous state (DCIS) or at a tumor stage where no metastases at all (neither proximal nor distal), i.e., Tᵢₛ, N0, M0 or T1-4; N0; M0 are present. Tᵢₛ denotes carcinoma *in situ.* In a preferred embodiment the detection of ErbB-3 is used to diagnose BC in a non-metastatic stage, i.e., that diagnosis is made at stage Tᵢₛ, N0, M0 or T1-3; N0; M0 (=Tᵢₛ,-3; N0;M0).

Whole blood, serum, plasma are the most widely used sources of sample in clinical routine. The identification of an early BC tumor marker that would allow reliable cancer detection or provide early prognostic information could lead to a diagnostic assay that would greatly aid in the diagnosis and in the management of this disease. Therefore, an urgent clinical need exists to improve the diagnosis of BC from blood. It is especially important to improve the early diagnosis of BC, since for patients diagnosed early on chances of survival are much higher as compared to those diagnosed at a progressed stage of disease.

It was the object of the present invention to investigate whether a biochemical marker can be identified which may be used in assessing cancer disease. In particular, the inventors of the present invention investigated whether a biochemical marker could be identified for the assessment of different cancer types, such as breast, colorectal, and/or ovarian cancer in tissue samples or body fluids.

Surprisingly, it has been found that use of the s-ErbB-3, comprising (i) the shedded extracellular domain of a human "c-erbB-3 oncogene" protein and (ii) the secreted protein isoforms encoded by splice variants of the mRNA derived from the human "c-erbB-3 oncogene", as biomarker can at least partially overcome some of the problems of the markers presently known in the state of the art.

### Summary of the Invention

In one embodiment the present invention relates to a method for assessing breast cancer in vitro comprising measuring in a serum or plasma sample the concentration of (a) s-ErbB-3, (b) optionally one or more other marker of cancer, and (c) using the measurement result of step (a) and optionally of step (b) in the assessment of breast cancer, wherein an increased concentration of a s-ErbB-3 is indicative for breast cancer.

Further the present invention relates to the use of s-ErbB-3 in the assessment of breast cancer in a serum or plasma sample.

Further the present invention relates to the use of an antibody directed against s-ErbB-3 protein in the in vitro assessment of breast cancer in a serum or plasma sample, wherein an increased concentration of s-ErbB-3 is indicative for breast cancer.

Further the present invention discloses the use of a marker panel comprising s-ErbB-3 and optionally one or more other marker for cancer in the in vitro assessment of breast cancer in a serum or plasma sample, wherein an increased concentration of s-ErbB-3 is indicative for breast cancer.

### Detailed Description of the Invention

In a preferred embodiment the present invention relates to a method for assessing breast cancer in vitro comprising measuring in a serum or plasma sample the concentration of s-ErbB-3 and using the measurement results, particularly the concentration determined in the assessment of breast cancer.

Surprisingly, it has been found that an increased concentration of s-ErbB-3 in the test sample is associated with the occurrence of cancer. It could be shown that s-ErbB-3 is a marker which is not specific for a single type of cancer, but a marker for different types of cancer, i.e. a general tumor marker. Since s-ErbB-3 appears to be rather specific for tumorigenic processes, the novel tumor marker s-ErbB-3 has great potential to be of clinical utility with various classes of tumor types.

Further a method of the present invention is suitable for the assessment of many different types of cancer. Increased concentrations of s-ErbB-3 in a sample as compared to normal controls have been found for example in specific cancer types like breast, colorectal and/or ovarian cancer, respectively.

According to a preferred embodiment of the invention, the concentration of s-ErbB-3 is measured in a sample in order to assess specific cancer types, such as breast, colorectal and/or ovarian cancer in vitro.

According to another preferred embodiment of the invention, the concentration of s-ErbB-3 is measured in a sample in order to assess cancer, such as breast, colorectal and/or ovarian cancer in vitro.

According to another preferred embodiment of the invention, the concentration of s-ErbB-3 is measured in a sample in order to assess cancer, such as breast and/or colorectal cancer in vitro.

According to another preferred embodiment of the invention, the concentration of s-ErbB-3 is measured in a sample in order to assess breast cancer in vitro.

One embodiment of the present invention refers to the mass screening of a population to distinguish between individuals which are probably free from cancer and individuals which might be classified as "suspect" cases. The latter group of individuals could then be subjected to further diagnostic procedures, e.g. by imaging methods or other suitable means.

A further embodiment of the present invention refers to an improvement of tumor marker panels which are suitable for the diagnosis of cancer in general or tumor marker panels which are suitable for the diagnosis of a specific tumor type, e.g. breast cancer.

The present invention is also directed to a method for assessing cancer in vitro by biochemical markers, comprising measuring in a sample the concentration of s-ErbB-3 and of one or more other markers specific for cancer, and using the measurement results, particularly the concentrations, determined in the assessment of cancer. Preferred markers for use in combination with s-ErbB-3 are, on the one hand, markers which are general tumor markers (i.e. markers which are not specific for a single tumor type) or, on the other hand, specific tumor markers (markers which are specific for a single tumor type). Preferred markers, e.g. for the assessment of cancer, such as breast cancer or colorectal cancer, are CYFRA 21-1, CEA, CA 15-3, CA 19-9 and ErbB-2 (HER-2). These markers may be used individually each or in any combination together with s-ErbB-3.

If, according to this method of the invention, cancer is assessed, the one or more other marker of the respective cancer is preferably selected from the group consisting of CYFRA 21-1, CEA, CA 15-3, CA 19-9 and ErbB-2.

Hence, the present invention, in a preferred embodiment, relates to the use of a marker panel comprising at least the marker s-ErbB-3 and at least one other tumor marker, e.g. of, in the assessment of cancer, e.g. breast, ovary and/or colorectal cancer.

The present invention also relates to the use of an antibody directed against s-ErbB-3 in the assessment of cancer, wherein an increased concentration of s-ErbB-3 is indicative for cancer.

Further the present invention relates to the use of an antibody directed against the secreted protein isoforms encoded by splice variants of the mRNA derived from the human "c-erbB-3 oncogene" in the assessment of cancer, wherein an increased concentration of the secreted protein isoforms encoded by splice variants of the mRNA derived from the human "c-erbB-3 oncogene" is indicative for cancer.

Further the present invention relates to the use of an antibody directed against the shedded extracellular domain of a human "c-erbB-3 oncogene" protein in the assessment of cancer, wherein an increased concentration of the shedded extracellular domain of a human "c-erbB-3 oncogene" protein is indicative for cancer.

Preferably, the present invention is directed to a method for assessing cancer, such as lung cancer or colorectal cancer in vitro by biochemical markers, comprising measuring in a sample the concentration of s-ErbB-3 and of one or more other cancer markers, e.g. one or more other markers of breast or colorectal cancer and using the measurement results, particularly concentrations determined in the assessment of cancer. It is preferred that the one or more other marker is selected from the group consisting of CYFRA 21-1, CEA, CA 15-3, CA 19-9 and ErbB-2.

The present invention, in a preferred embodiment, also relates to the use of a marker panel comprising at least s-ErbB-3 and CYFRA 21-1 in the assessment of cancer, particularly breast or colorectal cancer, and more particularly colorectal cancer.

The present invention, in a preferred embodiment, also relates to the use of a marker panel comprising at least s-ErbB-3 and CEA in the assessment of cancer, particularly breast or colorectal cancer, and more particularly colorectal cancer.

The present invention, in a preferred embodiment, also relates to the use of a marker panel comprising at least s-ErbB-3 and CA 15-3 in the assessment of cancer, particularly breast or colorectal cancer, and more particularly colorectal cancer.

The present invention, in a preferred embodiment, also relates to the use of a marker panel comprising at least s-ErbB-3 and CA 19-9 in the assessment of cancer, particularly breast or colorectal cancer, and more particularly colorectal cancer.

The present invention, in a preferred embodiment, also relates to the use of a marker panel comprising at least s-ErbB-3 and ErbB-2 in the assessment of cancer, particularly breast or colorectal cancer, and more particularly colorectal cancer. The present invention also relates to the use of an s-ErbB-3 protein in the assessment of cancer, wherein an increased concentration of s-ErbB-3 is indicative for cancer.

The present invention also relates to the use of s-ErbB-3 in the assessment of several specific types of cancer, particularly breast, colorectal and/or ovarian cancer.

In a preferred embodiment the present invention relates to a method for assessing breast cancer in vitro comprising measuring in a serum or plasma sample the concentration of a) s-ErbB-3, b) optionally one or more other marker of cancer, and (c) using the measurement results of step (a) and optionally of step (b) in the assessment of breast cancer, wherein an increased concentration of s-ErbB-3 is indicative for breast cancer.

The term "measurement" preferably comprises a qualitative, semi-qualitative or a quantitative measurement of s-ErbB-3 in a sample. In a preferred embodiment the measurement is a semi-quantitative measurement, i.e. it is determined whether the concentration of s-ErbB-3 is above or below a cut-off value. As the skilled artisan will appreciate, in a Yes- (presence) or No- (absence) assay, the assay sensitivity is usually set to match the cut-off value. A cut-off value can for example be determined from the testing of a group of healthy individuals. Preferably the cut-off is set to result in a specificity of 90%, also preferred the cut-off is set to result in a specificity of 95%, or also preferred the cut-off is set to result in a specificity of 98%. A value above the cut-off value can for example be indicative for the presence of cancer. In particular a value above the cut-off value can for example be indicative for the presence of breast, colorectal and/or ovarian cancer. In a further preferred embodiment the measurement of s-ErbB-3 is a quantitative measurement. In further embodiments the concentration of s-ErbB-3 is correlated to an underlying diagnostic question like e.g. stage of disease, disease progression, or response to therapy.

In certain other preferred embodiment, e.g. in monitoring of therapy or follow-up, the cut-off is set to result in a sensitivity of 90%, also preferred the cut-off is set to result in a sensitivity of 95%, or also preferred the cut-off is set to result in a sensitivity of 98%.

A value below the cut-off value can for example be indicative for the absence of cancer. In particular a value below the cut-off value can for example be indicative for the absence of breast, colorectal and/or ovarian cancer.

In a further preferred embodiment the measurement of s-ErbB-3 is a quantitative measurement. In further embodiments the concentration of s-ErbB-3 is correlated to an underlying diagnostic question like e.g. stage of disease, disease progression, or response to therapy.

ErbB-3, as well as ErbB1, ErbB2 and ErbB4, belongs to the epidermal growth factor receptor (EGFR/ErbB) family.

Upon binding to neuregulins, which are also known as differentiation factors or heuregulins, ErbB-3 becomes activated by heterodimerization with ErbB1, ErbB2 or ErbB4 (Riese, D.J. et al., Mol. Cell Biol. 15 (1995) 5770-5776). Through binding to ErbB-3 or ErbB4, neuregulins induce proliferation or differentiation of epithelial, glial, and muscle cells (Lee, H. et al., Oncogene 16 (1998) 3243-3252; Lee, H. et al, Cancer Res. 61 (2001) 4467-4473; Citri, A. et al., Exp. Cell Res. 284 (2003) 54-65).

The human ErbB-3 protein, which is also known as p180 ErbB-3 or "human epidermal growth factor receptor 3", is coded by the human c-erbB-3 oncogene. Said p180 ErbB-3 glycoprotein (180 kDa), encoded in the full-length mRNA derived from the human "c-erbB-3 oncogene", consists of an extracellular domain (ligand binding domain, ECD), a transmembrane domain and a cytoplasmic domain with homology to tyrosine kinases (Kraus, M.H. et al., PNAS 86 (1989) 9193-9197; Plowman, G.D. et al., PNAS 87 (1990) 4905-4909).

Elevated levels of secreted forms of ErbB3 in bone marrow and plasma samples of men with prostate cancer were found by Sue-Hwa, L. et al., Clin. Cancer Res. 14 (2008) 3729-3736.

Siegel, P.M. et al., EMBL J. 18 (1999), 2149-2164 have shown an elevated expression of NEU/ErbB2 and ErbB3 to be involved in the induction of mammary tumors in transgenic mice.

Immunoprecipitation analysis of primary cultures of human ovarian carcinomas shows the expression of a secreted 90kDa ErbB3 protein (Lee, H. et al., Oncogene 16 (1998) 3243-3252).

In US2008/274115 methods of detection of soluble ErbB3 and antibodies therefore have been disclosed. The authors found in normal and breast cancer tissue p85-s-ErbB3, which indicates a moderate to high expression of s-ErbB3.

The term "s-ErbB-3" relates to (i) the shedded extracellular domain of a human "c-erbB-3 oncogene" protein and (ii) the secreted protein isoforms encoded by splice variants of the mRNA derived from the human "c-erbB-3 oncogene".

Lee, H. et al., Oncogene 16 (1998) 3243-3252 have reported four new alternative c-erbB-3 oncogene transcripts (mRNAs) isolated from ovarian carcinoma derived cell line. The expression of these alternative isoforms (for details see below) were examined by using Northern Blot analysis wherein tissue and cell specific localization were observed (Katoh, M. et al., Biochem. Biophys. Res. Commun. 192 (1993) 1189-1197).

The shedded extracellular domain of the human p 180 ErbB-3 protein is shown in SEQ ID NO:1 (wildtype). The secreted protein isoforms encoded by splice variants of mRNAs derived from the human "c-erbB-3 oncogene" known today are shown in SEQ ID NO:2 (p45 ErbB-3, Isoform R2), SEQ ID NO:3 (p85 ErbB-3, Isoform R31), SEQ ID NO:4 (p85 ErbB-3, Isoform R35) and SEQ ID NO:5 (Isoform R1/VariantS).

Stably transfected fibroblast show that four truncated forms are soluble secreted proteins. In the culture medium of a primary ovarian cell line a 90 kDa ErbB-3 protein isoform was detectable by using an antibody against the ligand binding domain of ErbB-3 (The 90 kDa ErbB-3 protein isoform is a higher glycosylated form of the 85 kDa ErbB-3 protein isoform shown above). Additional studies suggest that secreted p85 ErbB-3 protein isoform inhibited the binding of neuregulin to the ErbB-3 receptor, which has the result that the endogenous ligand becomes neutralized. Together, this observations suggest that the p85 ErbB-3 protein isoform is a naturally occurring negative regulator of HRG-stimulated signal transduction. Recently, the secreted p45 ErbB-3 protein isoform has been identified in bone marrow supernatant samples from men with prostate cancer. In addition, immunhistochemical analysis of human tissue specimens, that the secreted p45 ErbB-3 protein isoform was highly expressed in metatstatic prostate cancer cells in bone (Chen, N. et al., Cancer Res. 67 (2007) 6544-6548).

As obvious to the person skilled in the art, the present invention shall not be construed to be limited to the full-length shedded extracellular domain of the ErbB-3 protein shown in SEQ ID NO:1 or the secreted protein isoforms encoded by splice variants of the mRNA derived from the human "c-erbB-3 oncogene" shown in SEQ ID NO:2 to 5. Physiological or artificial fragments of s-ErbB-3, secondary modifications of s-ErbB-3, as well as allelic variants of s-ErbB-3 are also encompassed by the present invention. Variants of a polypeptide are encoded by the same gene, but may differ in their isoelectric point (=PI) or molecular weight (=MW), or both e.g., as a result of alternative mRNA or pre-mRNA processing. The amino acid sequence of a variant is to 95% or more identical to the corresponding marker sequence. Artificial fragments preferably encompass a peptide produced synthetically or by recombinant techniques, which at least comprises one epitope of diagnostic interest consisting of at least 6, 7, 8, 9 or 10 contiguous amino acids as derived from the sequences disclosed in SEQ ID NO:1 to 5. Such fragment may advantageously be used for generation of antibodies or as a standard in an immunoassay. More preferred the artificial fragment comprises at least two epitopes of interest appropriate for setting up a sandwich immunoassay.

R&D Systems offers an ErbB-3 ELISA assay to measure natural and recombinant human epidermal growth factor receptor 3 in cell culture supernatants.

Genentech Inc. disclosed antibodies which bind to ErbB-3 protein and reduce HRG-induced formation of an ErbB2-ErbB-3 protein complex in a cell which expresses ErbB2 and ErbB-3. Further, antibodies which increase the binding affinity of heregulin for ErbB-3 protein and the characteristic of reducing HRG-induced ErbB2 activation in a cell which expresses ErbB2 and ErbB-3.

As used herein, each of the following terms has the meaning associated with it in this section.

The articles "a" and "an" are used herein to refer to one or to more than one (i.e. to at least one) of the grammatical object of the article. By way of example, "a marker" means one marker or more than one marker. The term "at least" is used to indicate that optionally one or more further objects may be present. By way of example, a marker panel comprising at least (the markers) s-ErbB-3 and CYFRA 21-1 may optionally comprise one or more other marker.

The expression "one or more" denotes 1 to 50, preferably 1 to 20 also preferred 2, 3, 4, 5, 6, 7, 8, 9, 10, 12, or 15.

The term "marker" or "biochemical marker" as used herein refers to a molecule to be used as a target for analyzing a patient's test sample. Examples of such molecular targets are proteins or polypeptides. Proteins or polypeptides used as a marker in the present invention are contemplated to include naturally occurring variants of said protein as well as fragments of said protein or said variant, in particular, immunologically detectable fragments. Immunologically detectable fragments preferably comprise at least 6, 7, 8, 10, 12, 15 or 20 contiguous amino acids of said marker polypeptide. One of skill in the art would recognize that proteins which are released by cells or present in the extracellular matrix may be damaged, e.g., during inflammation, and could become degraded or cleaved into such fragments. Certain markers are synthesized in an inactive form, which may be subsequently activated by proteolysis. As the skilled artisan will appreciate, proteins or fragments thereof may also be present as part of a complex. Such complex also may be used as a marker in the sense of the present invention. In addition, or in the alternative a marker polypeptide or a variant thereof may carry a post-translational modification. Non-limiting examples for posttranslational modifications are glycosylation, acylation, and/or phosphorylation.

s-ErbB-3 proteins, particularly (i) the shedded extracellular domain of a human "c-erbB-3 oncogene" protein and (ii) the secreted protein isoforms encoded by splice variants of the mRNA derived from the human "c-erbB-3 oncogene", are detected in appropriate samples. Preferred samples are tissue samples or body fluids, such as blood, plasma, serum, feces (preferred in the case of suspected CRC), nipple aspirate fluid (= NAF; preferred in the case of suspected BC) etc. Preferably, the sample is derived from a human subject, e.g. a tumor patient or a person in risk of a tumor or a person suspected of having a tumor. Also preferred s-ErbB-3 is detected in a serum or plasma sample.

In a preferred embodiment according to the present invention, the concentration of s-ErbB-3 is determined. In one embodiment, the marker s-ErbB-3 is specifically measured from a sample by use of a specific binding agent.

A specific binding agent is, e.g., a receptor for s-ErbB-3, a lectin binding to s-ErbB-3 or an antibody to s-ErbB-3. A specific binding agent has at least an affinity of 10⁷ l/mol for its corresponding target molecule. The specific binding agent preferably has an affinity of 10⁸ l/mol or also preferred of 10⁹ l/mol for its target molecule. As the skilled artisan will appreciate the term specific is used to indicate that other biomolecules present in the sample do not significantly bind to the binding agent specific for s-ErbB-3. Preferably, the level of binding to a biomolecule other than the target molecule results in a binding affinity which is at most only 10% or less, only 5% or less only 2% or less or only 1% or less of the affinity to the target molecule, respectively. A preferred specific binding agent will fulfil both the above minimum criteria for affinity as well as for specificity.

A specific binding agent preferably is an antibody reactive with s-ErbB-3. The term antibody refers to a polyclonal antibody, a monoclonal antibody, antigen binding fragments of such antibodies, single chain antibodies as well as to genetic constructs comprising the binding domain of an antibody.

Any antibody fragment retaining the above criteria of a specific binding agent can be used. Antibodies are generated by state of the art procedures, e.g., as described in Tijssen (Tijssen, P., Practice and theory of enzyme immunoassays, 11, Elsevier Science Publishers B.V., Amsterdam, the whole book, especially pages 43-78). In addition, the skilled artisan is well aware of methods based on immunosorbents that can be used for the specific isolation of antibodies. By these means the quality of polyclonal antibodies and hence their performance in immunoassays can be enhanced (Tijssen, P., supra, pages 108-115).

For the achievements as disclosed in the present invention polyclonal antibodies raised in rabbits may be used. However, clearly also polyclonal antibodies from different species, e.g., sheep or goat, as well as monoclonal antibodies can also be used. Since monoclonal antibodies can be produced in any amount required with constant properties, they represent ideal tools in development of an assay for clinical routine. The generation and the use of monoclonal antibodies to s-ErbB-3 in a method according to the present invention, respectively, represent yet other preferred embodiments.

As the skilled artisan will appreciate now that s-ErbB-3 has been identified as a marker which is useful in the assessment of cancer, preferably breast or colorectal cancer, various immunodiagnostic procedures may be used to reach a result comparable to the achievements of the present invention. For example, alternative strategies to generate antibodies may be used. Such strategies comprise amongst others the use of synthetic peptides, representing an epitope of s-ErbB-3 for immunization. Alternatively, DNA immunization also known as DNA vaccination may be used.

For measurement the sample obtained from an individual is incubated with the specific binding agent for s-ErbB-3 under conditions appropriate for formation of a binding agent s-ErbB-3 complex. Such conditions need not be specified, since the skilled artisan without any inventive effort can easily identify such appropriate incubation conditions. The amount of binding agent s-ErbB-3 complex is measured and used in the assessment of cancer, preferably of lung cancer. As the skilled artisan will appreciate there are numerous methods to measure the amount of the specific binding agent s-ErbB-3 complex all described in detail in relevant textbooks (cf., e.g., Tijssen P., supra, or Diamandis, E.P. and Christopoulos, T.K. (eds.), Immunoassay, Academic Press, Boston (1996)).

Preferably, s-ErbB-3 is detected in a sandwich-type assay format. In such assay, a first specific binding agent is used to capture s-ErbB-3 on the one side and a second specific binding agent, which is labeled to be directly or indirectly detectable, is used on the other side. The specific binding agents used in a sandwich-type assay format may be antibodies specifically directed against s-ErbB-3. The detection may be carried out by using different capturing and labeled antibodies, i.e. antibodies which recognize different epitopes on the s-ErbB-3 protein.

A "marker of cancer" and in particular a "marker of cancer selected from the group consisting of BC, CRC and OC" in the sense of the present invention is any marker that if combined with the marker s-ErbB-3 adds relevant information in the assessment of cancer disease in the assessment of cancer in general or in the assessment of certain cancer types, e.g. in the assessment of BC. The information is considered relevant or of additive value if at a given specificity the sensitivity, or if at a given sensitivity the specificity, respectively, for the assessment of cancer can be improved by including said marker into a marker combination already comprising the marker s-ErbB-3. In the preferred embodiment of cancer assessment, the improvement in sensitivity or specificity, respectively, is statistically significant at a level of significance of p = .05, .02, .01 or lower. Preferably, the one or more other tumor marker is selected from the group consisting of CYFRA 21-1, CEA, CA 15-3, CA 19-9 and ErbB-2.

The term "sample" as used herein refers to a biological sample obtained for the purpose of evaluation in vitro. In the methods of the present invention, the sample or patient sample preferably may comprise any body fluid. Preferred samples are whole blood, serum, plasma, nipple aspirate fluid (= NAF; preferred in the case of suspected BC), feces (preferred in the case of suspected CRC), tissue lysates or tissue samples, with plasma or serum being most preferred.

The term "tissue sample" and/or "tissue section" as used herein refers to a biological sample taken from a patient during surgery, therapeutic resections or a biopsy (e.g. incisional biopsy, excisional biopsy, core biopsy or needle aspiration biopsy) involving the removal of cells or tissues for the purpose of evaluation in vitro. When performing an analysis according to the present invention, the tissue sample material is used either directly or as a "tissue lysate". A "tissue sample" as used herein refers also to thin tissue slices usually accomplished through the use of a microtome. In any disclosed method embodiment involving a biological sample, such biological sample can be (but is not necessarily) mounted on a microscope slide, is a tissue section (such as a formalin-fixed and paraffin-embedded tissue section), and/or is a neoplastic tissue (such as, a lung cancer, colorectal cancer, head and neck cancer, gastric cancer, or glioblastoma).

A "tissue lysate", "cell lysate", "lysate", "lysed sample", "tissue extract" or "cell extract" as used herein refers to a sample and/or biological sample material comprising lysed tissue or cells, i.e. wherein the structural integrity of tissue or cells has been disrupted. To release the contents of cells or a tissue sample, the material is usually treated with enzymes and/or with chemicals to dissolve, degrade or disrupt the cellular walls and cellular membranes of such tissues or cells. The skilled artisan is fully familiar with appropriate methods for obtaining lysates. This process is encompassed by the term "lysis".

The term "assessing cancer" and in particular "assessing a cancer selected from the group consisting of BC, OC and CRC" is used to indicate that the method according to the present invention will (alone or together with other markers or variables, e.g., the criteria set forth by the UICC (see above)) e.g., aid the physician to establish or confirm the absence or presence of cancer, in particular of BC or aid the physician in the prognosis, the detection of recurrence (follow-up of patients after surgery) and/or the monitoring of treatment, especially of chemotherapy.

As the skilled artisan will appreciate, any such assessment is made in vitro. The patient sample is discarded afterwards. The patient sample is solely used for the in vitro diagnostic method of the invention and the material of the patient sample is not transferred back into the patient's body. Preferably, the sample is a liquid sample, e.g., whole blood, serum, or plasma.

Unless otherwise noted, technical terms are used according to conventional usage. Definitions of common terms in cell and molecular biology may be found in Lewin, B., Genes V, published by Oxford University Press (1994), ISBN 0-19-854287 9); Kendrew, J. et al. (eds.), The Encyclopedia of Molecular Biology, published by Blackwell Science Ltd. (1994), ISBN 0-632-02182-9; and Meyers, R.A. (ed.), Molecular Biology and Biotechnology: a Comprehensive Desk Reference, published by VCH Publishers, Inc. (1995), ISBN 1-56081-569 8.

In a preferred embodiment the present invention relates to a method for assessing cancer, e.g. BC, in vitro by biochemical markers, comprising measuring in a sample the concentration of s-ErbB-3 and using the concentration determined in the assessment of cancer, e.g. BC.

In another preferred embodiment the present invention relates to a method for assessing BC in vitro by biochemical markers, comprising measuring in a sample the concentration of s-ErbB-3 and using the concentration determined in the assessment of BC.

The inventors of the present invention have surprisingly been able to detect an increased concentration of the marker s-ErbB-3 in a significant percentage of samples derived from patients with cancer, in particular with breast cancer (BC), colorectal cancer (CRC) or ovarian cancer (OC). Even more surprising they have been able to demonstrate that the increased concentration of s-ErbB-3 in such sample obtained from an individual can be used in the assessment of cancer, in particular of the above-mentioned cancer diseases.

The ideal scenario for diagnosis would be a situation wherein a single event or process would cause the respective disease as, e.g., in infectious diseases. In all other cases correct diagnosis can be very difficult, especially when the etiology of the disease is not fully understood as is the case for many cancer types, e.g. for BC, CRC or OC. As the skilled artisan will appreciate, no biochemical marker is diagnostic with 100% specificity and at the same time 100% sensitivity for a given multifactorial disease, for example for BC. Rather, biochemical markers, e.g., CYFRA 21-1, CEA, CA 15-3, CA 19-9, ErbB-2, or as shown here s-ErbB-3 can be used to assess with a certain likelihood or predictive value e.g., the presence, absence, or the severity of a disease. Therefore in routine clinical diagnosis, generally various clinical symptoms and biological markers are considered together in the diagnosis, treatment and management of the underlying disease. Biochemical markers can either be determined individually or in a preferred embodiment of the invention they can be measured simultaneously using a chip or a bead based array technology. The concentrations of the biomarkers are then either interpreted independently, e.g., using an individual cut-off for each marker, or they are combined for interpretation.

In a further preferred embodiment the assessment of cancer according to the present invention is performed in a method comprising measuring in a sample the concentration of a) s-ErbB-3, b) one or more other marker of cancer, and c) using the measurement result, e.g. the concentration determined in step (a) and step (b), respectively, in the assessment of cancer.

In the assessment of cancer the marker s-ErbB-3 will be of advantage in one or more of the following aspects: screening; diagnostic aid; prognosis; monitoring of therapy such as chemotherapy, radiotherapy, and immunotherapy.

### Screening:

Screening is defined as the systematic application of a test to identify individuals e.g. at risk individuals, for indicators of a disease, e.g., the presence of cancer.
Preferably the screening population is composed of individuals known to be at higher than average risk of cancer. For example, a screening population for breast cancer is composed of individuals known to be at higher than average risk of cancer.

In the preferred embodiment, a tissue sample or any body fluid such as whole blood, plasma, serum, feces (preferred in the case of suspected CRC) or nipple aspirate fluid (= NAF; preferred in the case of suspected BC) is used in the screening for cancer, e.g. breast cancer.

In another preferred embodiment plasma, serum or NAF (preferred in the case of suspected BC) is used as a sample in the screening for breast cancer.

For many diseases, no single biochemical marker in the circulation will ever meet the sensitivity and specificity criteria required for screening purposes. This appears to be also true for cancer and in particular for breast cancer. It has to be expected that a marker panel comprising a plurality of markers will have to be used in cancer screening. The data established in the present invention indicate that with advantage the marker s-ErbB-3 forms an integral part of a marker panel suitable for screening purposes. The present invention therefore relates to the use of s-ErbB-3 as one marker of a cancer marker panel, i.e. a marker panel comprising s-ErbB-3 and one or more additional marker for cancer screening purposes. In particular, the present invention relates to the use of s-ErbB-3 as one marker of a general cancer marker panel. Such marker panel comprises the marker s-ErbB-3 and one or more additional markers, e.g. general cancer markers and/or markers for the above-mentioned type of cancer.

s-ErbB-3 is also likely to contribute to marker panels for certain specific types of cancer, e.g breast, colorectal and/or ovarian cancer.

Other preferred types of cancer to be assessed with a marker panel comprising s-ErbB-3 are breast, colorectal or ovarian cancer.

Other preferred types of cancer to be assessed with a marker panel comprising s-ErbB-3 are breast or colorectal cancer.

A preferred type of cancer to be assessed with a marker panel comprising s-ErbB-3 is breast cancer (BC).

The present data further indicate that certain combinations of markers will be advantageous in the screening for cancer. For example, with reference to the preferred embodiment of screening BC, OC or CRC, the present invention also relates to the use of a marker panel comprising s-ErbB-3 and CYFRA 21-1, or of a marker panel comprising s-ErbB-3 and CEA, or of a marker panel comprising s-ErbB-3 and CA 15-3, or of a marker panel comprising s-ErbB-3 and CA 19-9, or of a marker panel comprising s-ErbB-3 and ErbB-2, or of a marker panel comprising s-ErbB-3 and two or more markers selected from the group consisting of CYFRA 21-1, CEA, CA 15-3, CA 19-9 and ErbB-2.

### Diagnostic aid:

Markers may either aid the differential diagnosis of benign vs. malignant disease in a particular organ, help to distinguish between different histological types of a tumor, or to establish baseline marker values before surgery.

Today, important methods used in the detection of breast cancer are radiology and/or computed tomography (CT) scans. Small nodules, i.e. small regions of suspect tissue can be visualized by these methods. However, many of these nodules - more than 90% with CT - represent benign tissues changes, and only a minority of nodules represents cancerous tissue. Use of the marker s-ErbB-3 may aid in the differentiation of benign versus malign disease.

In a preferred embodiment the marker s-ErbB-3 is used in an immunohistological method in order to establish or confirm different histological types of BC, OC and/or CRC, preferably BC.

Since s-ErbB-3 as a single marker might be superior to other markers, e.g. in the case of BC to other markers, like CEA or CYFRA 21-1, it has to be expected that s-ErbB-3 will be used as a diagnostic aid, especially by establishing a baseline value before surgery. The present invention thus also relates to the use of s-ErbB-3 for establishing a baseline value before surgery for cancer.

### Prognosis:

Prognostic indicators can be defined as clinical, pathological, or biochemical features of cancer patients and their tumors that predict with a certain likelihood the disease outcome. Their main use is to help to rationally plan patient management, i.e. to avoid undertreatment of aggressive disease and overtreatment of indolent disease, respectively. Molina, R. et al., Tumor Biol. 24 (2003) 209-218 evaluated the prognostic value of CEA, CA 125, CYFRA 21-1, SSC and NSE in NSCLC. In their study abnormal serum levels of the markers NSE, CEA, and LDH (lactate dehydrogenase) appeared to indicate shorter survival.

As s-ErbB-3 alone significantly contributes to the differentiation of cancer patients, e.g. BC or CRC patients, from healthy controls, it has to be expected that it will aid in assessing the prognosis of patients suffering from cancer, preferably from BC or CRC. The level of preoperative s-ErbB-3 will most likely be combined with one or more other marker for cancer and/or the TNM staging system. In a preferred embodiment s-ErbB-3 is used in the prognosis of patients with BC, CRC, or OC.

### Monitoring of Therapy:

Merle, P. et al., Int. J. of Biological Markers 19 (2004) 310-315 have evaluated CYFRA 21-1 serum level variations in patients with locally advanced NSCLC (= non-small cell lung cancer) treated with induction chemotherapy. They conclude that early monitoring of CYFRA 21-1 serum levels may be a useful prognostic tool for tumor response and survival in stage III NSCLC patients. In addition, reports have described the use of CEA in monitoring the treatment of patients with LC (Fukasawa, T. et al., Gan to Kagku Ryoho 13 (1986) 1862-1867) Most of these were retrospective, non-randomized and contained small numbers of patients. As in the case of the studies with CYFRA 21-1 the CEA studies suggested: a) that patients with a decrease in CEA levels while receiving chemotherapy generally had a better outcome than those patients whose CEA levels failed to decrease and (b) for almost all patients, increases in CEA levels were associated with disease progression.

It is expected that s-ErbB-3 will be at least as good as a marker for monitoring of chemotherapy as CYFRA 21-1 or CEA, respectively. The present invention therefore also relates to the use of s-ErbB-3 in the monitoring of cancer patients and preferably of breast cancer (BC) or colorectal cancer (CRC) patients under chemotherapy. In the monitoring of therapy in one preferred embodiment the measurements for s-ErbB-3 and for at least one marker selected from the group consisting of CYFRA 21-1, CEA, CA 15-3, CA 19-9 and ErbB-2 will be combined and used in the assessment of BC or CRC.

### Follow-up:

A large portion of patients (e.g. lung cancer patients) who undergo surgical resection aimed at complete removal of cancerous tissue, later develop recurrent or metastatic disease (Wagner, H., Chest 117 (2000) 110-118; Buccheri, G. et al., Ann. Thorac. Surg. 75 (2003) 973-980). Most of these relapses occur within the first 2-3 years after surgery. Since recurrent/metastatic disease is invariably fatal if detected too late, considerable research has focused on cancer relapse at an early and thus potentially treatable stage.

Consequently, many cancer patients, e.g. BC patients undergo a postoperative surveillance program which frequently includes regular monitoring with CEA. Serial monitoring with CEA one year after surgical resection has been shown to detect an early postoperative recurrent/metastatic disease with a sensitivity of approximately 29 %, at a specificity of approximately 97 %, even in the absence of suspicious symptoms or signs (Buccheri, G. et al., Ann. Thorac. Surg. 75 (2003) 973-980). Thus, the follow-up of patients with BC after surgery is one of the most important fields of use for an appropriate biochemical marker. Due to the high sensitivity of s-ErbB-3 in the BC patients investigated it is likely that s-ErbB-3 alone or in combination with one or more other marker will be of great help in the follow-up of BC patients, especially in BC patients after surgery. The use of a marker panel comprising s-ErbB-3 and one or more other marker of BC in the follow-up of BC patients represents a further preferred embodiment of the present invention.

The present invention in a preferred embodiment relates to the use of s-ErbB-3 in the diagnostic field of cancer. Preferably s-ErbB-3 is used in the assessment of breast, colorectal or ovarian cancer, respectively.

In yet a further preferred embodiment the present invention relates to the use of s-ErbB-3 as a marker molecule for cancer, e.g. for cancer in general or for specific types of cancer, such as breast, colorectal or ovarian cancer in combination with one or more further marker molecules for cancer. The further marker molecules may be cancer-type unspecific general marker molecules and/or cancer-type specific marker molecules, e.g. marker molecules for breast, colorectal or ovarian cancer. s-ErbB-3 and the at least one further marker are used in the assessment of cancer, e.g. BC or CRC in a liquid sample obtained from an individual. Preferred selected other cancer markers with which the measurement of s-ErbB-3 may be combined are CYFRA 21-1, CEA, CA 15-3, CA 19-9 and/or ErbB-2. In particular, preferred selected other BC markers with which the measurement of s-ErbB-3 may be combined are CYFRA 21-1, CEA, CA 15-3, CA 19-9 and/or ErbB-2. Yet further preferred the marker panel used in the assessment of BC comprises s-ErbB-3 and at least one other marker molecule selected from the group consisting of CYFRA 21-1 and CEA.

As the skilled artisan will appreciate there are many ways to use the measurements of two or more markers in order to improve the diagnostic question under investigation. In a quite simple, but nonetheless often effective approach, a positive result is assumed if a sample is positive for at least one of the markers investigated. This may e.g. the case when diagnosing an infectious disease, like AIDS.

Frequently, however, the combination of markers is evaluated. Preferably the values measured for markers of a marker panel, e.g. for s-ErbB-3 and CYFRA 21-1, are mathematically combined and the combined value is correlated to the underlying diagnostic question. Marker values may be combined by any appropriate state of the art mathematical method. Well-known mathematical methods for correlating a marker combination to a disease employ methods like, discriminant analysis (DA) (i.e. linear-, quadratic-, regularized-DA), Kernel Methods (i.e. SVM), Nonparametric Methods (i.e. k-Nearest-Neighbor Classifiers), PLS (Partial Least Squares), Tree-Based Methods (i.e. Logic Regression, CART, Random Forest Methods, Boosting/Bagging Methods), Generalized Linear Models (i.e. Logistic Regression), Principal Components based Methods (i.e. SIMCA), Generalized Additive Models, Fuzzy Logic based Methods, Neural Networks and Genetic Algorithms based Methods. The skilled artisan will have no problem in selecting an appropriate method to evaluate a marker combination of the present invention. Preferably the method used in correlating the marker combination of the invention e.g. to the absence or presence of LC is selected from DA (i.e. Linear-, Quadratic-, Regularized Discriminant Analysis), Kernel Methods (i.e. SVM), Nonparametric Methods (i.e. k-Nearest-Neighbor Classifiers), PLS (Partial Least Squares), Tree-Based Methods (i.e. Logic Regression, CART, Random Forest Methods, Boosting Methods), or Generalized Linear Models (i.e. Logistic Regression). Details relating to these statistical methods are found in the following references: Ruczinski, I. et al., J. of Computational and Graphical Statistics 12 (2003) 475-511; Friedman, J.H., J. of the American Statistical Association 84 (1989) 165-175; Hastie, T. et al., The Elements of Statistical Learning, Springer Series in Statistics (2001); Breiman, L. et al., Classification and regression trees, Wadsworth, Inc., California (1984); Breiman, L., Random Forests, Machine Learning 45 (2001) 5-32; Pepe, M.S., The Statistical Evaluation of Medical Tests for Classification and Prediction, Oxford Statistical Science Series, 28 (2003); and Duda, R.O. et al., Pattern Classification, Wiley Interscience, 2nd edition (2001).

It is a preferred embodiment of the invention to use an optimized multivariate cut-off for the underlying combination of biological markers and to discriminate state A from state B, e.g. diseased from healthy. In this type of analysis the markers are no longer independent but form a marker panel.

Accuracy of a diagnostic method is best described by its receiver-operating characteristics (ROC) (see especially Zweig, M. H., and Campbell, G., Clin. Chem. 39 (1993) 561-577). The ROC graph is a plot of all of the sensitivity/specificity pairs resulting from continuously varying the decision thresh-hold over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease or benign versus malignant disease.

In each case, the ROC plot depicts the overlap between the two distributions by plotting the sensitivity versus 1 - specificity for the complete range of decision thresholds. On the y-axis is sensitivity, or the true-positive fraction [defined as (number of true-positive test results)/(number of true-positive + number of false-negative test results)]. This has also been referred to as positivity in the presence of a disease or condition. It is calculated solely from the affected subgroup. On the x-axis is the false-positive fraction, or 1 - specificity [defined as (number of false-positive results)/(number of true-negative + number of false-positive results)]. It is an index of specificity and is calculated entirely from the unaffected subgroup. Because the true- and false-positive fractions are calculated entirely separately, by using the test results from two different subgroups, the ROC plot is independent of the prevalence of disease in the sample. Each point on the ROC plot represents a sensitivity/1-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test.

One preferred way to quantify the diagnostic accuracy of a laboratory test is to express its performance by a single number. Such an overall parameter e.g. is the so-called "total error" or alternatively the "area under the curve = AUC". The most common global measure is the area under the ROC plot. By convention, this area is always ≥ 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

Combining measurements of s-ErbB-3 with other markers like CYFRA 21-1 or CEA, or with other markers of BC yet to be discovered, s-ErbB-3 leads and will lead, respectively, to further improvements in assessment of BC.

In a preferred embodiment the present invention relates to a method for improving the diagnostic accuracy for cancer, e.g. BC versus healthy controls by measuring in a sample the concentration of at least s-ErbB3 and CYFRA 21-1, and optionally of CEA, CA 15-3, CA 19-9 and/or HER-2, respectively and correlating the concentrations determined to the presence or absence of cancer, e.g. BC, the improvement resulting in more patients being correctly classified as suffering from cancer, e.g. BC versus healthy controls as compared to a classification based on any single marker investigated alone.

In a preferred method according to the present invention at least the concentration of the biomarkers s-ErbB-3 and CYFRA 21-1, respectively, is determined and the marker combination is used in the assessment of cancer, e.g. BC.

In another preferred method according to the present invention at least the concentration of the biomarkers s-ErbB-3 and CEA, respectively, is determined and the marker combination is used in the assessment of cancer, e.g. BC.

In another preferred method according to the present invention at least the concentration of the biomarkers s-ErbB-3 and CA 15-3, respectively, is determined and the marker combination is used in the assessment of cancer, e.g. BC.

In another preferred method according to the present invention at least the concentration of the biomarkers s-ErbB-3 and CA 19-9, respectively, is determined and the marker combination is used in the assessment of cancer, e.g. BC.

In another preferred method according to the present invention at least the concentration of the biomarkers s-ErbB-3 and ErbB-2, respectively, is determined and the marker combination is used in the assessment of cancer, e.g. BC.

In yet a further preferred method according to the present invention at least the concentration of the biomarkers s-ErbB-3, CYFRA 21-1 and CEA, respectively, is determined and the marker combination is used in the assessment of cancer, e.g. BC.

In yet another further preferred method according to the present invention at least the concentration of the biomarkers s-ErbB-3, CYFRA 21-1 and CA 15-3, respectively, is determined and the marker combination is used in the assessment of cancer, e.g. BC.

In yet another further preferred method according to the present invention at least the concentration of the biomarkers s-ErbB-3, CYFRA 21-1 and CA 19-9, respectively, is determined and the marker combination is used in the assessment of cancer, e.g. BC.

In yet another further preferred method according to the present invention at least the concentration of the biomarkers s-ErbB-3, CYFRA 21-1 and ErbB-2, respectively, is determined and the marker combination is used in the assessment of cancer, e.g. BC.

The following examples, sequence listing and figures are provided to aid the understanding of the present invention, the true scope of which is set forth in the appended claims. It is understood that modifications can be made in the procedures set forth without departing from the spirit of the invention.

### Description of the Figures

- **Figure 1**: shows the Boxplot of s-ErbB-3 in different disease groups. BC = breast cancer; CRC = colorectal cancer; ctrl = control cohort.
- **Figure 2**: shows the plot of the receiver operator characteristics (ROC-plot) of s-ErbB-3 in breast cancer (BC) samples with an AUC of 0.79 for the assessment of 39 samples obtained from patients with BC as compared to 43 control samples obtained from obviously healthy individuals.
- **Figure 3**: shows the plot of the receiver operator characteristics (ROC-plot) of s-ErbB-3 in colorectal cancer (CRC) samples with an AUC of 0.79 for the assessment of 110 samples obtained from patients with BC as compared to 43 control samples obtained from obviously healthy individuals.

### Description of the Sequences

- **SEQ ID NO: 1**: shows the amino acid sequence of the shedded extracellular domain encoded in the full-length mRNA derived from the human "c-erbB-3 oncogene" (wildtype); SwissProt database accession number: P21860.
- **SEQ ID NO: 2**: shows the amino acid sequence encoded in a splice variant of the mRNA derived from the human "c-erbB-3 oncogene"; the isoform is also known in the literature as "p45 s-ErbB-3" or "Isoform R2".
- **SEQ ID NO: 3**: shows the amino acid sequence encoded in a splice variant of the mRNA derived from the human "c-erbB-3 oncogene"; the isoform is also known in the literature as "p85 s-ErbB-3" or "Isoform R31 ".
- **SEQ ID NO: 4**: shows the amino acid sequence encoded in a splice variant of the mRNA derived from the human "c-erbB-3 oncogene"; the isoform is also known in the literature as "p85 s-ErbB-3" or "Isoform R35".
- **SEQ ID NO: 5**: shows the amino acid sequence encoded in a splice variant of the mRNA derived from the human "c-erbB-3 oncogene"; the isoform is also known in the literature as "Isoform R1/VariantS".
- **SEQ ID NO: 6**: shows an amino acid motif of the juxtamembrane (JM) region of the human p180 ErbB-3 (HER-3) glycoprotein.
- **SEQ ID NO: 7**: shows an amino acid motif of the juxtamembrane (JM) region of the human ErbB-2 (HER-2).
- **SEQ ID NO: 8**: Primer 1 (sense)
- **SEQ ID NO: 9**: Primer 2 (antisense)
- **SEQ ID NO: 10**: Primer 3 (antisense)
- **SEQ ID NO: 11**: Primer 4 (antisense)

### Example 1

### Identification of s-ErbB-3 as a potential marker for breast cancer

It is known, that the extracellular domain of HER-2/neu, a 185kDa transmembrane protein, undergoes proteolytical cleavage by metalloproteases and is shed into blood as a circulating antigen. Serum HER-2 has been shown to have prognostic and predictive information in breast cancer. The transmembrane receptor HER-2 form together with HER-3 a high affinity heregulin co-receptor (Sliwkowski, M.X. et al., J. Biol. Chem. 269 (1994) 14661-14665) which is believed to elicit potent mitogenic and transforming signal.

In addition a motif in the juxtamembrane (JM) region of EGF receptor family is believed where the region contains only 11 amino residues in which cleavage positions exist. The motif of the cleavage position is defines by a motif of proline/glycine residues with 5-7 amino acid residues in between (P/GX 5-7 P/G). Proline and glycine residues are known to disrupt secondary structures locally and are found in known cleavage sites. In the protein sequence of human full length p180 ErbB-3 protein (HER-3wt) this motif can also be found in the juxtamembrane region:
**Human HER-2 (ErbB-2)** juxtamembrane region (SEQ ID NO: 7)
**Human HER-3 (ErbB-3)** juxtamembrane region (SEQ ID NO: 6)

Serum samples from cancer patients but also from apparently healthy people (control cohort) were measured for level of s-ErbB-3 protein by using ELISA. Cross reactivity with other EGF family members is exclude by testing the antibody specificity with recombinant extracellular domains of HER-1, HER-2 and HER-4. No cross reactivity is observed.

The amino acid sequence of the extracellular domain of the human p180 ErbB-3 protein encoded in the full-length mRNA derived from the human "c-erbB-3 oncogene" is shown in SEQ ID NO:1. Translation of splice variant mRNA's derived from the human "c-erbB-3 oncogene" result in proteins shown in SEQ ID NO:2 (p45 s-ErbB-3, Isoform R2), SEQ ID NO:3 (p85 s-ErbB-3, Isoform R31), SEQ ID NO:4 (p85 s-ErbB-3, Isoform R35) and SEQ ID NO:5 (Isoform R1/VariantS). All proteins were expressed in mammalian cells HEK 293T and tested by Western Blot and ELISA.

### Example 2

### Antibodies to the breast cancer marker protein s-ErbB-3

Monoclonal antibodies to the cancer marker protein s-ErbB-3 are purchased from R&D Systems (Cat. No. DY348) for measurement of serum and plasma and blood levels of s-ErbB-3 by immunodetection assays, e.g. Western Blotting and ELISA.

### Recombinant protein expression in HEK 293 T cells

In order to generate calibration material to s-ErbB-3 protein, recombinant expression of wildtype (wt) p180 ErbB-3 and alternate transcript (mRNA) of ErbB-3 protein is performed. The expression is done in mammalian cells HEK 293T. In a first step, PCR is performed with oligonucleotide primers specific for a portion of the published nucleotide sequence of human c-erbB-3 gene as described (Lee, H. et al., Oncogene 16 (1998) 3243-3252). The nucleotide sequences of the synthesized primers are selected as follow:
Primer 1 (sense) SEQ ID NO: 8
   5'- gcaagctagccaccatgagggcgaacgacgctctgc -3'
Primer 2 (antisense) SEQ ID NO: 9
   5'- gcaagcggccgccccacctttgggacatagtcccccacaaggc -3'
Primer 3 (antisense) SEQ ID NO: 10
   5'- gcaagcggccgcttgtatgccacctgaacagttccattgcag -3'
Primer 4 (antisense) SEQ ID NO: 11
   5'- gcaagcggccgcacacccccttcctccttggttccatccctc -3'

The PCR products were cloned into tagged mammalian expression vector pCMV-Fc (Clonetech), and sequenced by Geneart.

Cell line: The human embryonic kidney cell line 293 was cultured in DMEM supplemented with 10% fetal calf serum.

### Purification of s-ErbB-3 protein:

All s-ErbB-3 proteins were isolated from the concentrated medium of cells transiently transfected with clone isoform R2, isoform R35, isoform R31 or isoform R1/VariantS and were purified in one step. Using a HiTrap chromatography with a Protein A column (Amersham), the protein was loaded over night at 4°C to the column. Bound material has been was washed with buffer A (10 mM phosphate, pH 7.4, 27mM KCl and 137 mM NaCl) incubated with PreScission protease over night at 4°C, and then eluted using buffer B containing 0.1M sodium citrate (pH 3.5). GST tagged PreScission protease was removed by using a GSTrap (1ml) column. Samples taken from each step were subjected to SDS-PAGE and analyzed by Coomassie Brilliant Blue staining and Western Blot using anti-ErbB-3 antibody recognizing the extracellular domain of s-ErbB-3.

### Example 3

### ELISA for the measurement of s-ErbB-3 protein in human serum and plasma samples or other body fluids

For detection of s-ErbB-3 protein in human serum or plasma, a sandwich ELISA is developed. For capture and detection of the antigen, aliquots of the anti-ErbB-3 monoclonal antibody (see example 2) are conjugated with biotin.

Streptavidin-coated 96-well microwell plates are incubated with 100 µl biotinylated anti-s-ErbB-3 polyclonal antibody overnight at 2 µg/ml in 10 mM phosphate, pH 7.4, 27mM KCl and 137 mM NaCl. After incubation, plates are washed three times with 10 mM phosphate, pH 7.4, 27mM KCl and 137 mM NaCl. Wells are then incubated for 2 h with 10 mM phosphate, pH 7.4, 1% BSA, 27mM KCl and 137 mM NaCl to block unspecific binding. After three times washing wells are incubated with either a serial dilution of the recombinant protein (see Example 2) as standard antigen or with diluted liquid samples obtained from patients. After binding of sErbB-3, plates are washed three times with 10 mM phosphate, pH 7.4, 27mM KCl and 137 mM NaCl. For specific detection of bound s-ErbB-3, wells are incubated with 100 µl of anti-s-ErbB-3 monoclonal antibody for 60 min at 4 µg/ml in 10 mM phosphate, pH 7.4, 1% BSA and 137mM NaCl. Thereafter, plates are washed three times to remove unbound antibody. In a next step, wells are incubated with 200µg/ml anti-mouse-POD conjugates for 30 min in 10 mM phosphate, pH 7.4, 1% BSA, 0.9% NaCl and 0.1 % Tween 20. Plates are subsequently washed three times with the same buffer. For detection of antigen-antibody complexes, wells are incubated with 100 µl ABTS solution (R&D Systems) and OD is measured after 30 min at 495 nm with an ELISA reader.

### Example 4

### ROC analysis to assess clinical utility in terms of diagnostic accuracy

Accuracy is assessed by analyzing individual liquid samples obtained from well-characterized patient cohorts, i.e., 50 patients having undergone mammography and found to be free of BC, 50 patients each diagnosed and staged as invasive ductal and invasive lobular T1-3, N0, M0 of BC, 50 patients diagnosed with progressed BC, having at least tumor infiltration in at least one proximal lymph node or more severe forms of metastasis, 50 patients each diagnosed with medullary, mucinous, tubular, or papillary breast carcinoma, and 50 patients diagnosed with DCIS, respectively. CA 15-3 as measured by a commercially available assay (Roche Diagnostics, CA 15-3-assay (Cat. No. 0 304 5838 for Elecsys^{®} Systems immunoassay analyzer) and s-ErbB-3 protein measured as described above have been quantified in a serum obtained from each of these individuals. ROC-analysis is performed according to Zweig, M. H., and Campbell, *supra.* Discriminatory power for differentiating patients in the group T1-3, N0, M0 from healthy individuals as measured by the area under the curve is found to be at least as good for the biomarker s-ErbB-3 as compared to the established marker CA 15-3.

### Measurement values of assay s-ErbB-3:

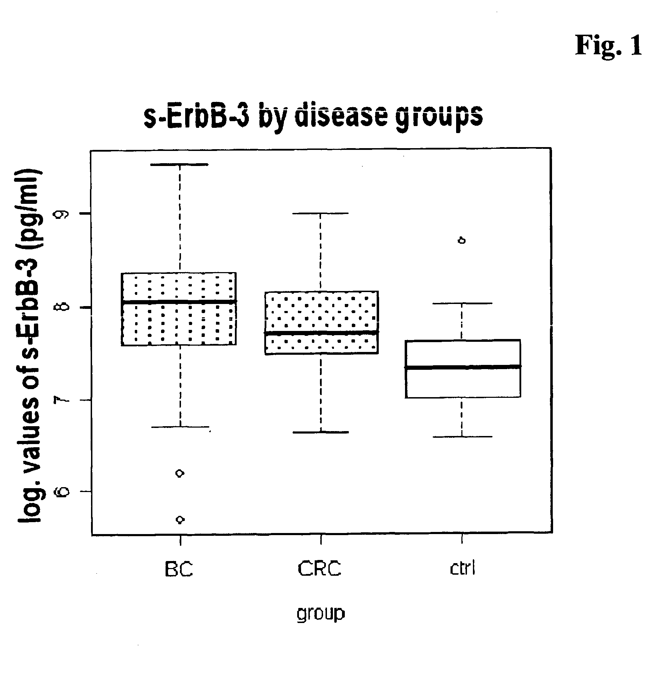

The cancer marker s-ErbB-3 concentration in human serum is measured in samples from breast cancer (BC) patients, colorectal cancer (CRC) patients and in a control cohort (Ctrl) of apparently healthy individuals. Table 1 shows the distribution of the absolute measurement values of s-ErbB-3 level in human serum of said groups by showing mean and median, minimum and maximum values, as well as the first and third quartile (25% and 75% percentile) of values. The data of Table 1 are shown in logarithmised form in Table 2 and as a diagram in the Boxplot of Figure 1.

### s-ErbB-3 as a serum marker for breast cancer (BC):

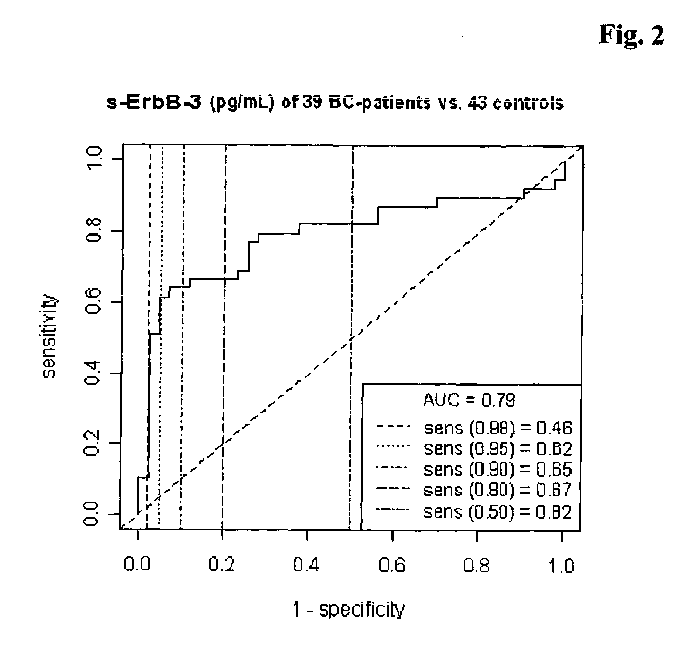

Samples derived from 39 well-characterized breast cancer (BC) patients with the data given in Table 3. Figure 2 shows receiver-operating characteristic curves (ROC) of s-ErbB-3 level in human serum. The marker has been determined in BC collective (39 patients) and 43 control samples obtained from obviously healthy individuals (= control cohort), resulting in an AUC of 0.79.

### s-ErbB-3 as_a serum marker for colorectal cancer (CRC):

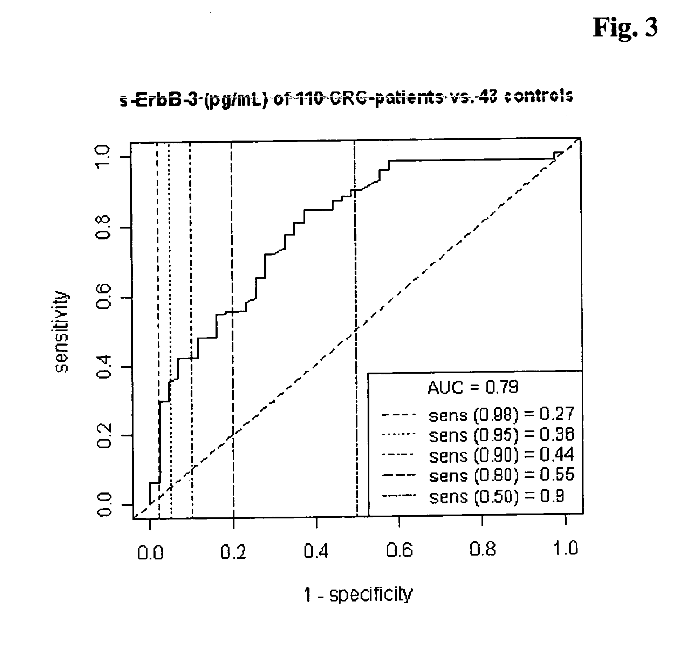

Samples derived from 110 well-characterized colorectal cancer (CRC) patients with the data given in Table 3. Figure 3 shows the receiver-operating characteristic curves (ROC) of s-ErbB-3 level in human serum. The marker has been determined in CRC collective (110 patients) and 43 control samples obtained from obviously healthy individuals (= control cohort), resulting in an AUC of 0.79.

### Summaries:

**Table 1:**

| **Distribution of absolute measurement values of s-ErbB-3 level in human serum of BC patients, CRC patients and control cohort:** | | | | | | | |
|---|---|---|---|---|---|---|---|
| | Min. | 1st Qu. | Median | Mean | 3rd Qu. | Max. | n |
| BC | 295,4 | 1974 | 3170 | 3748 | 4339 | 13620 | 39 |
| CRC | 753,2 | 1805 | 2249 | 2824 | 3459 | 8015 | 110 |
| Ctrl | 712,4 | 1109 | 1527 | 1680 | 2030 | 5914 | 43 |

| | | | | | | | |
|---|---|---|---|---|---|---|---|
| BC = breast cancer; CRC = colorectal cancer; Ctrl = control cohort; Min. = minimum of values; 1st Qu. = first quartile (25% percentile for values); 3rd Qu. = third quartile (75% percentile for values); Max. = maximum of values; n = number of patients | | | | | | | |

**Table 2:**

| **Distribution of logarithmised measurement values of s-ErbB-3 level in human serum of BC patients, CRC patients and control cohort:** | | | | | | | |
|---|---|---|---|---|---|---|---|
| | Min. | 1st Qu. | Median | Mean | 3rd Qu. | Max. | n |
| BC | 5,688 | 7,588 | 8,061 | 7,957 | 8,375 | 9,519 | 39 |
| CRC | 6,624 | 7,498 | 7,718 | 7,83 | 8,149 | 8,989 | 110 |
| Ctrl | 6,569 | 7,011 | 7,331 | 7,337 | 7,615 | 8,685 | 43 |

| | | | | | | | |
|---|---|---|---|---|---|---|---|
| BC = breast cancer; CRC = colorectal cancer; Ctrl = control cohort; Min. = minimum of values; 1st Qu. = first quartile (25% percentile for values); 3rd Qu. = third quartile (75% percentile for values); Max. = maximum of values; n = number of patients | | | | | | | |

The following data indicate that the cancer marker s-ErbB-3 is also helpful in the follow-up of patients after surgery.

**Table 3:**

| **Diagnostic Sensitivity and Specificity of s-ErbB-3 for breast cancer (BC) and colorectal cancer (CRC)** | | | |
|---|---|---|---|
| **Panel A** | CRC | BC | App. healthy ind. (Ctrl) |
| Antigen /Format | ErbB-3 R&D Systems ELISA assay | | |
| Cut-off value in pg/ml for a Specificity of 95% | > 2787 | > 3091 | < 3091 |
| Sensitivity | 33 % | 51 % | |
| No. of tested sera | 110 | 39 | 43 |

## Claims

1. A method for assessing breast cancer in vitro comprising measuring in a serum or plasma sample the concentration of
a) s-ErbB-3,
b) optionally one or more other marker of cancer, and
c) using the measurement result of step (a) and optionally of step (b) in the assessment of breast cancer, wherein an increased concentration of a s-ErbB-3 is indicative for breast cancer.

2. The method according to claim 1, further **characterized in that** said one or more other marker of step (b) is selected from the group consisting of CYFRA 21-1, CEA, CA 15-3, CA 19-9 and ErbB-2.

3. The method according any one of claims 1 to 2, further **characterized in that** the concentration is measured by an immunological method.

4. Use of s-ErbB-3 in the in vitro assessment of breast cancer in a serum or plasma sample.

5. Use of an antibody directed against s-ErbB-3 in the in vitro assessment of breast cancer in a serum or plasma sample, wherein an increased concentration of s-ErbB-3 is indicative for breast cancer.

6. The use of a marker panel comprising s-ErbB-3 and optionally one or more other marker for cancer in the in vitro assessment of breast cancer in a serum or plasma sample, wherein an increased concentration of s-ErbB-3 is indicative for breast cancer.

7. The use of the marker panel according to claim 6, further **characterized in that** the optionally one or more other marker is selected from the group consisting of CYFRA 21-1, CEA, CA 15-3, CA 19-9 and ErbB-2.

## Patentansprüche

1. Verfahren zur in-vitro-Diagnose von Brustkrebs, bei dem man in einer Serum- oder Plasmaprobe die Konzentration von
a) s-ErbB-3,
b) gegebenenfalls einem oder mehreren anderen Krebsmarkern misst und
c) die Messergebnisse aus Schritt (a) und gegebenenfalls Schritt (b) für die Diagnose von Brustkrebs verwendet, wobei eine erhöhte Konzentration an s-ErbB-3 indikativ für Brustkrebs ist.

2. Verfahren nach Anspruch 1, weiterhin **dadurch gekennzeichnet, dass** der eine oder die mehreren anderen Marker aus Schritt (b) aus der aus CYFRA 21-1, CEA, CA 15-3, CA 19-9 und ErbB-2 bestehenden Gruppe ausgewählt ist/sind.

3. Verfahren nach Anspruch 1 oder 2, weiterhin **dadurch gekennzeichnet, dass** die Konzentration durch eine immunologische Methode gemessen wird.

4. Verwendung von s-ErbB-3 zur in-vitro-Diagnose von Brustkrebs in einer Serum- oder Plasmaprobe.

5. Verwendung eines gegen s-ErbB-3 gerichteten Antikörpers bei der in-vitro-Diagnose von Brustkrebs in einer Serum- oder Plasmaprobe, wobei eine erhöhte Konzentration an s-ErbB-3 indikativ für Brustkrebs ist.

6. Verwendung eines s-ErbB-3 und gegebenenfalls einen oder mehrere andere Krebsmarker umfassenden Markerpanels zur in-vitro-Diagnose von Brustkrebs in einer Serum- oder Plasmaprobe, wobei eine erhöhte Konzentration an s-ErbB-3 indikativ für Brustkrebs ist.

7. Verwendung des Markerpanels nach Anspruch 6, weiterhin **dadurch gekennzeichnet, dass** der eine oder die mehreren gegebenenfalls gemessenen anderen Marker aus der aus CYFRA 21-1, CEA, CA 15-3, CA 19-9 und ErbB-2 bestehenden Gruppe ausgewählt ist/sind.

## Revendications

1. Procédé d'évaluation d'un cancer du sein *in vitro* comprenant la mesure dans un échantillon de sérum ou de plasma de la concentration de
a) s-ErbB-3,
b) facultativement un ou plusieurs autre(s) marqueur(s) de cancer, et
c) l'utilisation du résultat de mesure de l'étape (a) et facultativement de l'étape (b) dans l'évaluation d'un cancer du sein, dans lequel une concentration accrue d'un s-ErbB-3 est indicatrice d'un cancer du sein.

2. Procédé selon la revendication 1, **caractérisé en outre en ce que** ledit/lesdits un ou plusieurs autre(s) marqueur(s) de l'étape (b) est/sont choisi(s) parmi le groupe consistant en CYFRA 21-1, CEA, CA 15-3, CA 19-9 et ErbB-2.

3. Procédé selon l'une quelconque des revendications 1 à 2, **caractérisé en outre en ce que** la concentration est mesurée par un procédé immunologique.

4. Utilisation de s-ErbB-3 dans l'évaluation *in vitro* d'un cancer du sein dans un échantillon de sérum ou de plasma.

5. Utilisation d'un anticorps dirigé contre s-ErbB-3 dans l'évaluation *in vitro* d'un cancer du sein dans un échantillon de sérum ou de plasma, dans laquelle une concentration accrue de s-ErbB-3 est indicatrice d'un cancer du sein.

6. Utilisation d'un panel de marqueurs comprenant s-ErbB-3 et facultativement un ou plusieurs autre(s) marqueur(s) de cancer dans l'évaluation *in vitro* d'un cancer du sein dans un échantillon de sérum ou de plasma, dans laquelle une concentration accrue de s-ErbB-3 est indicatrice d'un cancer du sein.

7. Utilisation du panel de marqueurs selon la revendication 6, **caractérisée en outre en ce que** le/les un ou plusieurs autre(s) marqueur(s) facultatif(s) est/sont choisi(s) parmi le groupe consistant en CYFRA 21-1, CEA, CA 15-3, CA 19-9 et ErbB-2.
